# EUROPÄISCHE PATENTSCHRIFT

(11) **EP 1 268 446 B1**
(45) Veröffentlichungstag und Bekanntmachung des Hinweises auf die Patenterteilung: **06.10.2010**
(21) Anmeldenummer: 01915326.1
(22) Anmeldetag: 12.03.2001
(51) Int. Cl.: C07D 239/52, C07D 413/12, C07D 419/12

(54) **VERFAHREN ZUR HERSTELLUNG VON UNSYMMETRISCHEN 4,6-BIS(ARYLOXY)PYRIMIDIN-DERIVATEN**
METHOD FOR PRODUCING ASYMMETRICAL 4,6-BIS(ARYLOXY)PYRIMIDINE DERIVATIVES
PROCEDE DE PRODUCTION DE DERIVES ASYMETRIQUES DE 4,6-BIS(ARYLOXY)PYRIMIDINE

(30) Priorität: 24.03.2000 DE 10014607
(43) Veröffentlichungstag der Anmeldung: 02.01.2003
(73) Patentinhaber: Bayer CropScience AG, 40789 Monheim (DE)
(72) Erfinder: WEINTRITT, Holger, 40764 Langenfeld (DE); STELZER, Uwe, 51399 Burscheid (DE); GAYER, Herbert, 40789 Monheim (DE); HÜBSCH, Walter, 42113 Wuppertal (DE)
(86) Internationale Anmeldenummer: PCT/EP2001/002731
(87) Internationale Veröffentlichungsnummer: WO 2001/072719

(56) Entgegenhaltungen:
- EP-A- 0 794 177
- EP-A- 0 902 020
- WO-A-95/05367
- DE-A- 19 602 095

## Beschreibung

Die Erfindung betrifft ein neues Verfahren zur Herstellung von unsymmetrischen, bekannten 4,6-Bis(aryloxy)pyrimidin-Derivaten.

Unsymmetrische 4,6-Bis(aryloxy)pyrimidin-Derivate sind bekannt und werden beispielsweise im Pflanzenschutz als Schädlingsbekämpfungsmittel eingesetzt (vgl. WO 94/02470, WO 97/27189, WO 98/21189, WO 99/57116).

Die Herstellung unsymmetrischer 4,6-Bis(aryloxy)pyrimidin-Derivate ist schwieriger als die Herstellung symmetrischer 4,6-Bis(aryloxy)pyrimidin-Verbindungen, da die unterschiedlichen Aryloxygruppen in getrennten Reaktionen eingeführt werden müssen.

Es sind bereits mehrere Verfahren zur Herstellung von unsymmetrischen 4,6-Bis(aryloxy)pyrimidin-Derivaten bekannt.

In WO 94/02470 wird die Herstellung unsymmetrischer 4,6-Bis(aryloxy)pyrimidin-Derivate nach einem zweistufigen Verfahren beschrieben. Durch Umsetzung von 4,6-Dichlorpyrimidin (A) mit einem Äquivalent eines Phenolderivats (B) unter basischen Reaktionsbedingungen und anschließender Reaktion mit einem zweiten Phenolderivat (D) werden unsymmetrische 4,6-Bis(aryloxy)-pyrimidin-Derivate (E) erhalten (vgl. Schema 1).

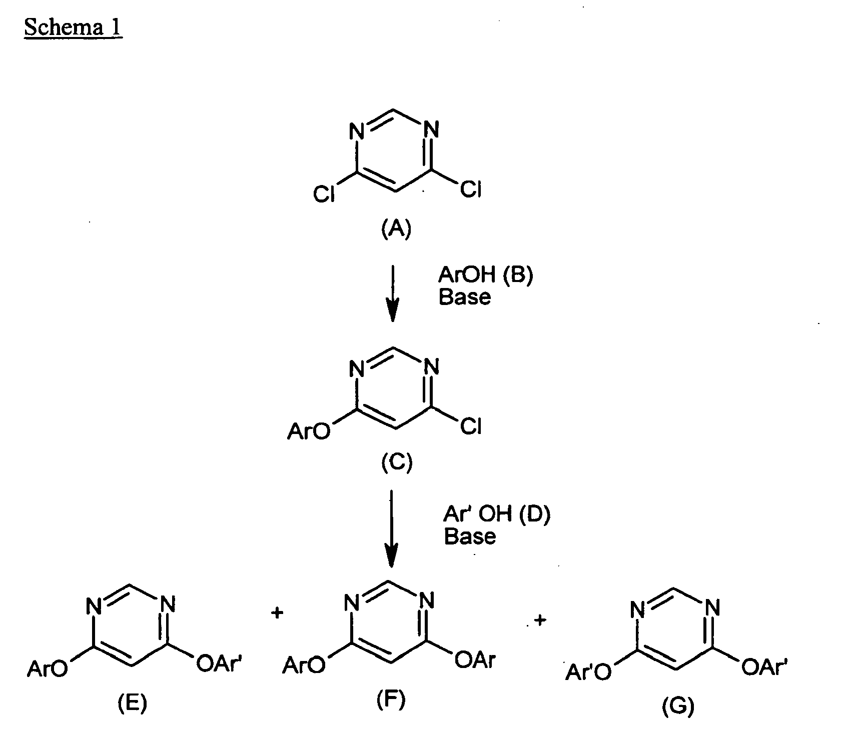

Der Nachteil dieses Verfahrens liegt darin, dass es im zweiten Reaktionsschritt zum Austausch der Aryloxygruppen kommt, wodurch ein Produktgemisch aus unsymmetrischen 4,6-Bis(aryloxy)pyrimidin-Derivaten (E) und den symmetrischen 4,6-Bis(aryloxy)pyrimidin-Derivaten (F) und (G) erhalten wird.

Die unsymmetrischen 4,6-Bis(aryloxy)pyrimidin-Derivate (E) werden daher in schlechter Ausbeute erhalten und können nur mittels aufwendiger Trennmethoden isoliert werden.

Um das Problem des Austauschs der Aryloxygruppen in der zweiten Reaktionstufe zu umgehen, kann als Ausgangsverbindung 4,6-Difluorpyrimidin verwendet werden (vgl. Schema 2 und WO 94/02470, EP-A1-794 177).

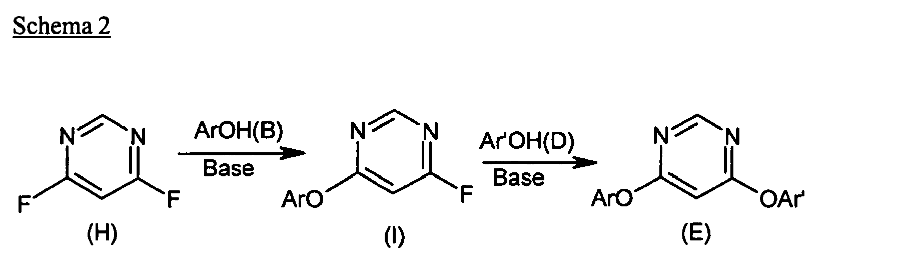

Ein Nachteil dieses Verfahrens liegt jedoch darin, dass 4,6-Difluorpyrimidin durch einen Chlor-Fluor-Austausch ausgehend von 4,6-Dichlor-pyrimidin hergestellt wird. Folglich ist zur Herstellung von unsymmetrischen 4,6-Bis(aryloxy)pyrimidin-Derivaten ein zusätzlicher Reaktionsschritt erforderlich. Daher sind 4,6-Dichlorpyrimidin bzw. 4,6-Dichlorpyrimidin-Derivate als Ausgangsverbindungen bevorzugt.

Die Herstellung von unsymmetrischen 4,6-Bis(aryloxy)pyrimidin-Derivaten, ausgehend von 4,6-Dichlor-5-halogen-pyrimidin analog dem in WO 94/02470 beschriebenen Verfahren wird in WO 98/41513 beschrieben.

In EP-A1-794 177, US 5,849,910 und US 5,977,363 wird ein weiteres Verfahren zur Herstellung von unsymmetrischen 4,6-Bis(aryloxy)pyrimidin-Derivaten (E) ausgehend von 4,6-Dichlorpyrimidin (A) beschrieben (vgl. Schema 3).

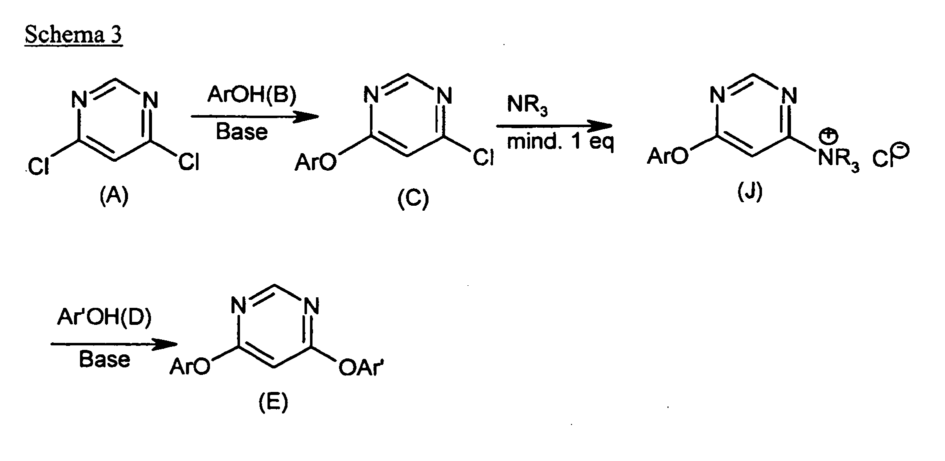

In diesem Verfahren wird das nach dem ersten Reaktionsschritt erhaltene Aryloxy-Chlorpyrimidin-Derivat (C) mit mindestens einem molaren Äquivalent eines tertiären Amins behandelt.

Als Zwischenprodukte entstehen Pyrimidinyl-Ammonium-Derivate (J), die mit Phenol-Derivaten (D) zu den unsymmetrischen 4,6-Bis(aryloxy)pyrimidin-Derivaten (E) umgesetzt werden.

Der Nachteil dieses Verfahrens liegt daran, dass das tertiäre Amin mindestens in molaren äquivalenten Mengen erforderlich ist und nur mittels aufwendiger Verfahren zurückgewonnen werden kann. Außerdem werden die unsymmetrischen 4,6-Bis(aryl-oxy)pyrimidin-Derivate nur in mäßigen Ausbeuten erhalten. Dieses Verfahren ist daher für die großtechnische Herstellung, vor allem bei Verwendung teurer Amine, nicht geeignet.

Es wurde nun gefunden, dass man unsymmetrische 4,6-Bis(aryloxy)pyrimidin-Derivate der allgemeinen Formel (I), 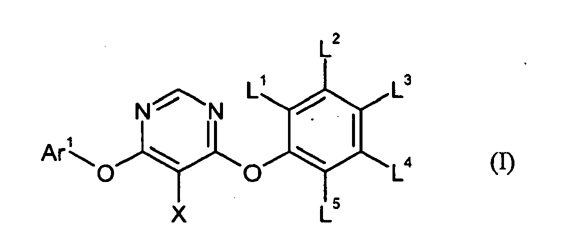 in welcher
Ar¹ für gegebenenfalls durch Halogen oder durch Alkyl, Alkoxy, Halogenalkyl, Halogenalkoxy mit jeweils 1 bis 4 Kohlenstoffatomen substituiertes Heterocyclyl mit 3 bis 7 Ringgliedern steht;
   oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Formyl, Carboxy, Carbamoyl, Thiocarbamoyl;
jeweils geradkettiges oder verzweigtes Alkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Dialkylamino,
Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl, mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung 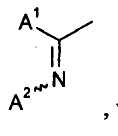 worin
A¹ für Wasserstoff, Hydroxy oder Alkyl mit 1 bis 4 Kohlenstoffatomen oder Cycloalkyl mit 1 bis 6 Kohlenstoffatomen steht und
A² für Hydroxy, Methoxy, Ethoxy, Amino, Methylamino, Phenyl, Benzyl oder für jeweils gegebenenfalls durch Cyano, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht,
   sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Benzoyl, Benzoylethenyl, Cinnamoyl, Heterocyclyl oder Phenylalkyl, Phenylalkyloxy oder Heterocyclylalkyl, mit jeweils 1 bis 3 Kohlenstoffatomen in den jeweiligen Alkylteilen,
X für Fluor oder Chlor steht,
L¹, L², L³, L⁴ und L⁵ gleich oder verschieden sind und unabhängig voneinander jeweils für Wasserstoff, Halogen, Cyano, Nitro, Formyl, Alkylcarbonyl, Alkoxycarbonyl, Aminocarbonyl, Alkylaminocarbonyl, Dialkylaminocarbonyl mit jeweils 1 bis 6 Kohlenstoffatomen, jeweils gegebenenfalls durch 1 bis 5 Halogenatome substituiertes Alkyl, Alkoxy, Alkylthio, Alkylsulfonyl oder Alkylsulfonyl mit jeweils 1 bis 6 Kohlenstoffatomen stehen,
   oder
L¹, L², L³ und L⁴ gleich oder verschieden sind und unabhängig voneinander jeweils für Wasserstoff, Halogen, Cyano, Nitro, Formyl, Alkylcarbonyl, Alkoxycarbonyl, Aminocarbonyl, Alkylaminocarbonyl, Dialkylaminocarbonyl, mit jeweils 1 bis 6 Kohlenstoffatomen, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen stehen und
   und
L⁵ für eine der folgenden Gruppen steht: 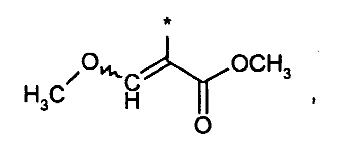 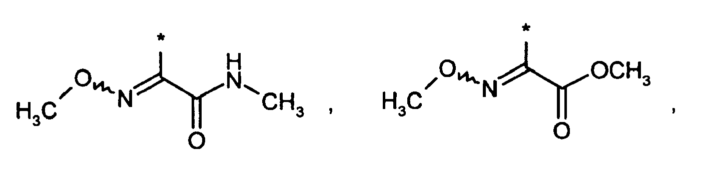 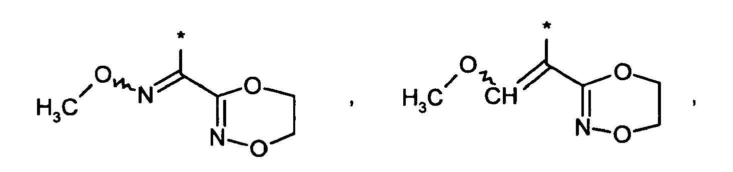 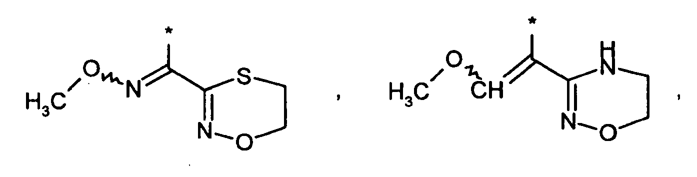 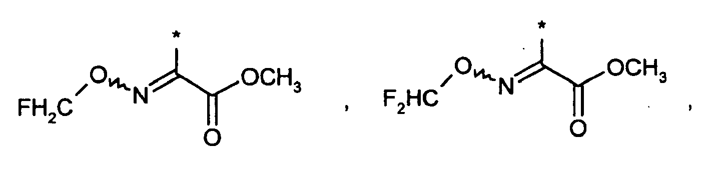 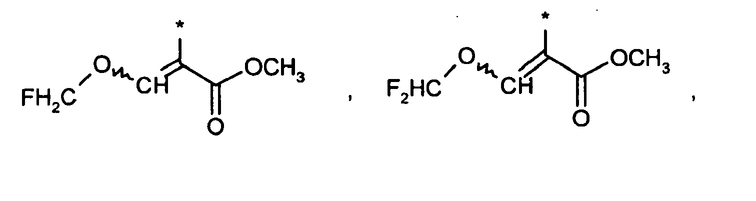 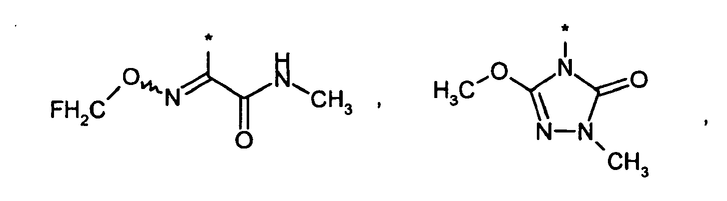 wobei * die Anknüpfungsstelle an den Phenylrest bezeichnet,
   und wobei die Reste
Ar¹ und 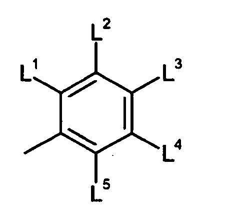 verschieden sind,
   erhält, wenn man
4,6-Dichlorpyrimidin-Derivate der allgemeinen Formel (II), 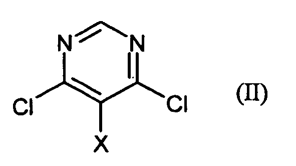 in welcher
X die oben angegebene Bedeutung hat,

a) zunächst in einer ersten Stufe mit Verbindungen der allgemeinen Formel (III),

   Ar¹-OH, (III)

   in welcher
   Ar¹ die oben angegebene Bedeutung hat,
   gegebenenfalls in Gegenwart eines Verdünnungsmittels und gegebenenfalls in Gegenwart eines Säureakzeptors umsetzt,
   und die dabei entstehenden Verbindungen der Formel (IV), 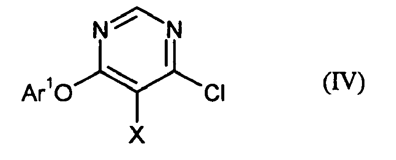 in welcher
   Ar¹ und X die oben angegebenen Bedeutungen haben,
      dann in einer zweiten Stufe mit Verbindungen der allgemeinen Formel (V), 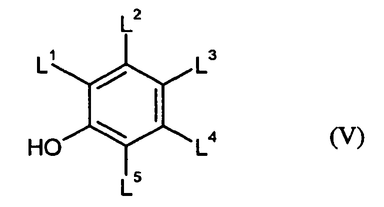 in welcher
   L¹, L², L³, L⁴ und L⁵ die oben angegebenen Bedeutungen haben,
   gegebenenfalls in Gegenwart eines Lösungsmittel, in Gegenwart einer Base und unter Zusatz von 2 bis 20 Mol-% 1,4-Diazabicyclo[2.2.2]octan (DABCO) umsetzt,
      oder
b) zunächst in einer ersten Stufe mit Verbindungen der allgemeinen Formel (V), 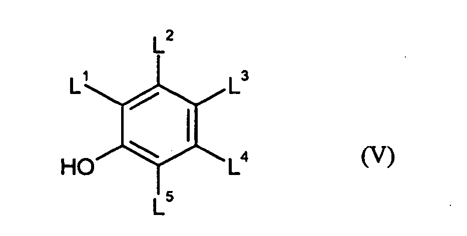 in welcher
   L¹, L², L³, L⁴ und L² die oben angegebenen Bedeutungen haben,
   gegebenenfalls in Gegenwart eines Verdünnungsmittels und gegebenenfalls in Gegenwart eines Säureakzeptors umsetzt,
   und die dabei entstehenden Verbindungen der Formel (VI), 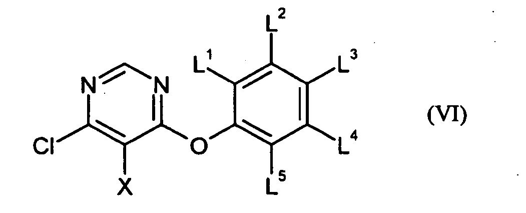 in welcher
   X, L¹, L², L³, L⁴ und L⁵ die oben angegebenen Bedeutungen haben,
      dann in einer zweiten Stufe mit Verbindungen der allgemeinen Formel (III),

      Ar¹-OH, (III)

      in welcher
   Ar¹ die oben angegebene Bedeutung hat,
   gegebenenfalls in Gegenwart eines Lösungsmittel, gegebenenfalls in Gegenwart einer Base und unter Zusatz von 2 bis 20 Mol-% 1,4-Diazabicyclo[2.2.2]octan (DABCO) umsetzt.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie beispielsweise in Alkoxy, Alkylthio oder Alkylamino, jeweils geradkettig oder verzweigt. Bevorzugt sind, wenn nicht anders angegeben, Kohlenwasserstoffketten mit 1 bis 6 Kohlenstoffatomen. Bevorzugt sind für Alkenyl oder Alkinyl, wenn nicht anders angegeben, Kohlenwasserstoffketten mit 2 bis 6 Kohlenstoffatomen.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono- oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Enthält der Ring mehrere Sauerstoffatome, stehen diese nicht benachbart. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Ein polycyclisches Ringsystem kann mit einem heterocyclischen Ring oder einem ankondensierten carbocyclischen Ring verknüpft sein. Das so beschriebene Heterocyclyl kann auch einfach oder mehrfach substituiert sein, vorzugsweise durch Methyl, Ethyl oder Halogen. Bevorzugt sind mono- oder bicyclische Ringsysteme, insbesondere mono- oder bicyclische Ringsysteme.

Halogenalkoxy steht für teilweise oder vollständig halogeniertes Alkoxy. Bei mehrfach halogeniertem Halogenalkoxy können die Halogenatome gleich oder verschieden sein. Bevorzugte Halogenatome sind Fluor und Chlor, insbesondere Fluor. Trägt das Halogenalkoxy noch weitere Substituenten, reduziert sich die maximal mögliche Zahl der Halogenatome auf die verschiedenen freien Valenzen. Bevorzugt sind, wenn nicht anders angegeben, Kohlenwasserstoffketten mit 1 bis 6 Kohlenstoffatomen.

Halogenalkyl steht für teilweise oder vollständig halogeniertes Alkyl. Bei mehrfach halogeniertem Halogenalkyl können die Halogenatome gleich oder verschieden sein. Bevorzugte Halogenatome sind Fluor und Chlor, insbesondere Fluor. Trägt das Halogenalkyl noch weitere Substituenten, reduziert sich die maximal mögliche Zahl der Halogenatome auf die verbleibenden freien Valenzen. Bevorzugt sind, wenn nicht anders angegeben, Kohlenwasserstoffketten mit 1 bis 6 Kohlenstoffatomen. Die Ausgangsverbindungen der Formeln (III) und (V), die Zwischenprodukte der Formeln (IV) und (VI) und die Endprodukte der allgemeinen Formel (I) können als reine Isomere verschiedener möglicher isomerer Formen, z. B. als E oder Z-Isomere oder gegebenenfalls als Mischungen verschiedener möglicher isomerer Formen, insbesondere von Heteroisomeren, wie z.B. als E-/Z-Gemische, vorliegen.

Bevorzugt sind Verbindungen, in denen Ar¹ für:
gegebenenfalls durch Methyl, Ethyl, Methoxy, Ethoxy, Trifluormethyl oder Trifluormethoxy substituiertes Thienyl, Pyridyl oder Furyl steht;
oder für jeweils gegebenenfalls einfach bis vierfach, gleich oder verschieden substituiertes Phenyl steht, wobei die möglichen Substituenten vorzugsweise aus der nachstehenden Aufzählung ausgewählt sind:
Fluor, Chlor, Brom, Iod, Cyano, Nitro, Formyl, Carboxy, Carbamoyl, Thiocarbamoyl,
Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), 3-Oxobutyl, Methoxymethyl, Dimethoxymethyl, Methoxy, Ethoxy, n- oder i-Propoxy,
Methylthio, Ethylthio, n- oder i-Propylthio, Methylsulfinyl, Ethylsulfinyl, Methylsulfonyl oder Ethylsulfonyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy,
oder eine Gruppierung 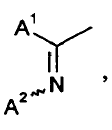 wobei
A¹ für Wasserstoff, Methyl oder Hydroxy steht und
A² für Hydroxy, Methoxy, Ethoxy, Amino, Methylamino, Phenyl oder Benzyl steht, sowie
   jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Benzoyl, Benzoylethenyl, Cinnamoyl, Benzyl, Phenylethyl, Phenylpropyl, Benzyloxy, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazolyl.

In einer weiteren ganz besonders bevorzugten Gruppe von Verbindungen steht Ar¹ für einfach bis vierfach substituiertes Phenyl, wobei die Substituenten aus der nachstehenden Aufzählung ausgewählt sind:
Halogen, Cyano, jeweils geradkettiges oder verzweigtes Alkyl oder Halogenalkyl mit insbesondere 1 bis 4 Kohlenstoffatomen.

Besonders bevorzugt sind Verbindungen, in denen X für Fluor steht.

Bevorzugt sind Verbindungen, in denen L¹, L², L³ und L⁴ gleich oder verschieden sind und unabhängig voneinander vorzugsweise jeweils für Wasserstoff, Fluor, Chlor, Brom, Cyano, Nitro, Acetyl, Propionyl, Methoxycarbonyl, Ethoxycarbonyl, Methylaminocarbonyl, Ethylaminocarbonyl, Dimethylaminocarbonyl, Diethylaminocarbonyl, Methyl, Ethyl, n- oder i-Propyl, n-, i-, s- oder t-Butyl, Methoxy, Ethoxy, n-oder i-Propoxy, Methylthio, Ethylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl stehen.

In einer ganz besonders bevorzugten Gruppe von Verbindungen stehen L¹, L², L³ und L⁴ für Wasserstoff oder Methyl.

In einer weiteren ganz besonders bevorzugten Gruppe von Verbindungen stehen stehen L¹, L², L³ und L⁴ für Wasserstoff.

Bevorzugt sind Verbindungen, in denen L⁵ für eine der folgenden Gruppen steht: 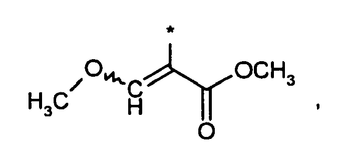 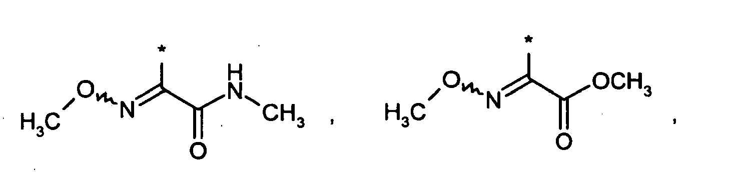 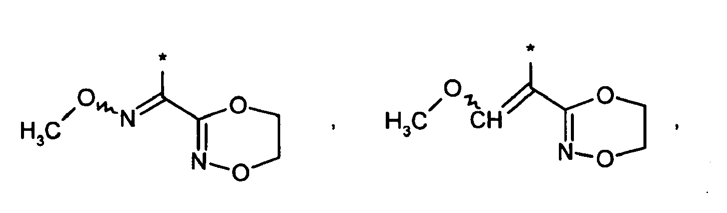 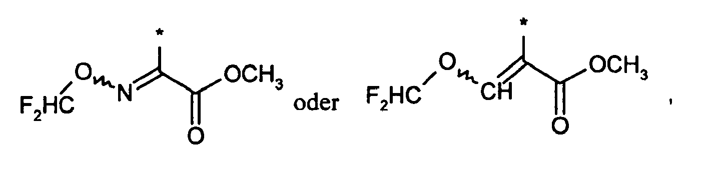 wobei * die Anknüpfungsstelle an den Phenylrest bezeichnet.

In einer ganz besonders bevorzugten Gruppe von Verbindungen steht L⁵ für eine der folgenden Gruppen: 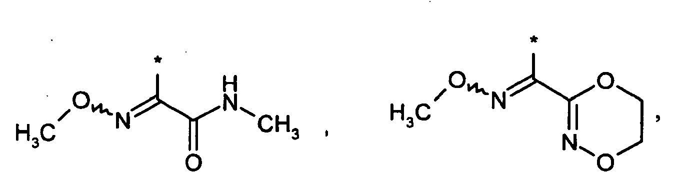 wobei * die Anknüpfungsstelle an den Phenylrest bezeichnet.

Die oben aufgeführten allgemein oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Reste angegebenen Restedefinitionen werden unabhängig von der jeweilig angegebenen Kombination der Reste, beliebig auch durch Restedefinitionen anderer Vorzugsbereiche ersetzt.

Es ist als ausgesprochen überraschend zu bezeichnen, dass beim erfindungsgemäßen Verfahren Aryloxyhalogenpyrimidin-Derivate unter Zusatz von 2 bis 20 Mol-% des tertiären Amins 1,4-Diazabicyclo[2.2.2]octan (DABCO) zu unsymmetrischen 4,6-Bis(aryl)oxypyrimidin-Derivaten mit hoher Selektivität und Ausbeute reagieren. Da im Stand der Technik (vgl. EP-A1-794 177, US 5,849,990 und US 5,977,363) darauf hingewiesen wird, dass für diese Reaktion mindestens ein molares Äquivalent eines tertiären Amins erforderlich ist, ist es äußerst überraschend, dass diese Reaktion auch mit 2 bis 20 Mol-% DABCO in hervorragenden Ausbeuten durchgeführt werden kann. Dies wird durch einen Vergleichsversuch bestätigt, bei dem die Umsetzung ohne Zusatz von DABCO durchgeführt wurde (vgl. Beispiel 4, zweite Stufe). Das Produkt kann nur in sehr schlechten Ausbeuten isoliert werden.

Das erfindungsgemäße Verfahren weist eine Reihe von Vorteilen auf. Die unsymmetrischen 4,6-Bis(aryloxy)pyrimidin-Derivate werden in hohen Ausbeuten und Reinheiten erhalten. Außerdem können als Ausgangsverbindungen die im Vergleich zu den 4,6-Difluorpyrimidin-Derivaten leichter zugänglichen 4,6-Dichlorpyrimidin-Derivate eingesetzt werden. Die Wiedergewinnung des Amins ist ebenfalls nicht erforderlich, da nur katalytische Mengen an DABCO zur Durchführung des Verfahrens erforderlich sind.

Die zur Durchführung des erfindungsgemäßen Verfahrens als Ausgangsstoffe benötigten Verbindungen der Formel (II) sind bekannt und lassen sich nach bekannten Methoden herstellen (vgl. DE-A1-197 10 609, WO 97/49605, DE-A1-196 42 533 und DE-A1- 195 31299) oder sind käufliche Handelsprodukte.

Die zur Durchführung des erfindungsgemäßen Verfahrens als Ausgangsstoffe benötigten Verbindungen der Formel (III) sind gängige Handelsprodukte oder können durch einfache Verfahren aus diesen hergestellt werden.

Die zur Durchführung des erfindungsgemäßen Verfahrens als Ausgangsstoffe benötigten Verbindungen der Formel (V) sind bekannt und lassen sich nach bekannten Methoden herstellen (vgl. DE-A1 196 11 653, WO-A-95/24396, WO 95/04728, WO 97/27189, WO 97/14687, WO 98/23155, WO 98/21189, WO 98/55461, WO 99/09026, WO 99/57116). Alle anderen Ausgangsverbindungen sind gängige Handelsprodukte oder können nach einfachen Verfahren aus diesen hergestellt werden.

Als Verdünnungsmittel zur Durchführung der ersten Stufe des erfindungsgemäßen Verfahrens kommen alle inerten organischen Lösungsmittel in Betracht. Hierzu gehören beispielhaft und vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe wie beispielsweise Chlorbenzol, Dichlorbenzol, Dichlormethan, Chloroform, Tetrachlormethan, Dichlorethan oder Trichlorethan; Ether wie beispielsweise Diethylether, Diisopropylether, Methyl-t-butylether, Methyl-t-amylether, Dioxan, Tetrahydrofuran, 1,2-Dimethoxyethan, 1,2-Diethoxyethan oder Anisol, Ketone wie beispielweise Aceton, Butanon, Methylisobutylketon oder Cyclohexanon, Nitrile, wie beispielsweise Acetonnitril, Propionitril, n- oder i-Butyronitril oder Benzonitril, Amide, wie N,N'-Dimethylformamid, N,N'-Dimethylacetamid, N-Methylformamid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; oder deren Gemische mit Wasser. Bevorzugt werden in der ersten Stufe des erfindungsgemäßen Verfahrens Ketone, insbesondere Methylisobutylketon verwendet.

Die erste Stufe des erfindungsgemäßen Verfahrens wird gegebenenfalls in Gegenwart eines geeigneten Säureakzeptors durchgeführt. Als solche kommen alle üblichen anorganischen oder organischen Basen infrage. Hierzu gehören beispielhaft und vorzugsweise Erdalkalimetall- oder Alkalimetallhydroxide, -acetate, -carbonate oder -hydrogencarbonate, wie beispielsweise Natriumhydroxid, Kaliumhydroxid, Natriumacetat, Kaliumacetat, Natriumcarbonat, Kaliumcarbonat, Kaliumhydrogencarbonat oder Natriumhydrogencarbonat; tertiäre Amine, wie beispielsweise Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclononen (DBN) oder Diazabicycloundecen (DBU); sowie Erdalkalimetall- oder Alkalimetallhydride, wie beispielsweise Calciumhydrid, Natrium- oder Kaliumhydrid. Bevorzugt werden in der ersten Stufe des erfindungsgemäßen Verfahrens Erdalkalimetall- oder Alkalimetallcarbonate, insbesondere Kalium- oder Natriumcarbonat verwendet.

Die Reaktionstemperaturen können bei der der ersten Stufe des erfindungsgemäßen Verfahrens in einem größerem Bereich variiert werden. Im Allgemeinen arbeitet man bei Temperaturen von 0°C oder 100°C, vorzugsweise bei Temperaturen von 40°C bis 80°C.

Zur Durchführung des erfindungsgemäßen Verfahrens setzt man pro Mol der Verbindungen der Formel (III) im Allgemeinen 1 bis 4 Mol, vorzugsweise 1 bis 1,1 Mol des 4,6-Dichlorpyrimidin-Derivates der Formel (II) ein.

Zur Durchführung des erfindungsgemäßen Verfahrens setzt man pro Mol der Verbindungen der Formel (V) im Allgemeinen 1 bis 4 Mol, vorzugsweise 1 bis 1,1 Mol des 4,6-Dichlorpyrimidin-Derivates der Formel (II) ein.

Zur Durchführung der ersten Stufe des erfindungsgemäßen Verfahrens geht man im Allgemeinen wie folgt vor. Das 4,6-Dichlorpyrimidin-Derivat der Formel (II) wird gegebenenfalls in Gegenwart eines Verdünnungsmittels mit einer Base versetzt. Hierzu gibt man die Verbindung der Formel (III) bzw. die Verbindung der Formel (V) gegebenenfalls in Gegenwart eines Verdünnungsmittels, und rührt gegebenenfalls bei erhöhter oder bei erniedrigter Temperatur, bis die Umsetzung vollständig ist. Nach beendeter Umsetzung wird das Reaktionsgemisch in üblicher Weise aufgearbeitet oder direkt in situ in der zweiten Stufe des erfindungsgemäßen Verfahrens umgesetzt.

Die Zugabe von Verbindungen der Formel (III) bzw. von Verbindungen der Formel (V) gegebenenfalls in Gegenwart eines Verdünnungsmittels in der ersten Stufe des erfindungsgemäßen Verfahrens erfolgt insbesondere durch Dosierung zu Verbindungen der Formel (II), die gegebenenfalls in einem Keton, insbesondere in Methylisobutylketon gelöst sind. Die Zugabe erfolgt auf einmal bis innerhalb von 12 Stunden, bevorzugt auf einmal bis innerhalb von 6 Stunden.

Als Verdünnungsmittel zur Durchführung der zweiten Stufe des erfindungsgemäßen Verfahrens kommen alle inerten organischen Lösungsmittel in Betracht. Hierzu gehören beispielhaft und vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie beispielsweise Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie beispielsweise Aceton, Butanon, Methylisobutylketon oder Cyclohexanon; Nitrile, wie beispielsweise Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; oder deren Gemische mit Wasser. Bevorzugt werden in der zweiten Stufe des erfindungsgemäßen Verfahrens Ketone, insbesondere Methylisobutylketon verwendet.

Die zweite Stufe des erfindungsgemäßen Verfahrens wird gegebenenfalls in Gegenwart eines geeigneten Säureakzeptors durchgeführt. Als solche kommen alle üblichen anorganischen oder organischen Basen infrage. Hierzu gehören beispielhaft und vorzugsweise Erdalkalimetall- oder Alkalimetallhydroxide, -acetate, -carbonate oder -hydrogencarbonate, wie Natriumhydroxid, Kaliumhydroxid, Natriumacetat, Kaliumacetat, Natriumcarbonat, Kaliumcarbonat, Kaliumhydrogencarbonat oder Natriumhydrogencarbonat; sowie Erdalkali- oder Alkalimetallhydride, wie beispielsweise Calciumhydrid, Natrium- oder Kaliumhydrid. Bevorzugt in der zweiten Stufe des erfindungsgemäßen Verfahrens Erdalkali- oder Alkalimetallcarbonate, insbesondere Kalium- oder Natriumcarbonat verwendet.

Die zweite Stufe des erfindungsgemäßen Verfahrens wird in Gegenwart von katalytischen Mengen 1,4-Diazabicyclo[2.2.2]octan (DABCO) durchgeführt.

Die Reaktionstemperaturen können bei der Durchführung zweiten Stufe des erfindungsgemäßen Verfahrens in einem größeren Bereich variert werden. Im Allgemeinen arbeitet man bei Temperaturen von 0°C bis 100°C, vorzugsweise bei Temperaturen von 40°C bis 90°, insbesondere bei Temperaturen von 50°C bis 80°C.

Zur Durchführung des erfindungsgemäßen Verfahrens setzt man pro Mol der Verbindungen der Formel (IV) im Allgemeinen 0,8 bis 4 Mol, vorzugsweise 0,95 bis 1,05 Mol der Verbindungen der Formel (V) ein.

Zur Durchführung des erfindungsgemäßen Verfahrens setzt man pro Mol der Verbindungen der Formel (VI) im Allgemeinen 0,8 bis 4 Mol, vorzugsweise 0,95 bis 1,05 Mol der Verbindungen der Formel (III) ein.

Zur Durchführung des erfindungsgemäßen Verfahrens setzt man pro Mol der Verbindungen der Formel (IV) im Allgemeinen 2 bis 20 Mol-% 1,4-Diazabicyclo[2.2.2]octan ein.

Zur Durchführung des erfindungsgemäßen Verfahrens setzt man pro Mol der Verbindungen der Formel (VI) im Allgemeinen 2 bis 20 Mol-%, bevorzugt 2 bis 20 Mol-% 1,4-Diazabicyclo[2.2.2]octan ein.
Zur Durchführung der zweiten Stufe der Verfahrensvariante a) geht man im Allgemeinen wie folgt vor. Die Verbindungen der Formel (V) werden gegebenenfalls in Gegenwart eines Verdünnungsmittels mit einer Base und 1,4-Diazabicyclo[2.2.2]-octan versetzt. Hierzu gibt man die Verbindungen der Formel (IV) gegebenenfalls in Gegenwart eines Verdünnungsmittels und rührt, gegebenenfalls bei erhöhter Temperatur. Nach beendeter Reaktion wird das Reaktionsgemisch in üblicher Weise aufgearbeitet.

Alternativ kann die Durchführung der zweiten Stufe der Verfahrensvariante a) auch dadurch erfolgen, dass die Verbindungen der Formel (IV) gegebenenfalls in Gegenwart eines Verdünnungsmittels mit einer Base und 1,4-Diazabicyclo[2.2.2]octan versetzt werden. Hierzu gibt man die Verbindungen der Formel (V) gegebenenfalls in Gegenwart eines Verdünnungsmittels und rührt, gegebenenfalls bei erhöhter Temperatur. Nach beendeter Reaktion wird das Reaktionsgemisch in üblicher Weise aufgearbeitet.

Zur Durchführung der zweiten Stufe der Verfahrensvariante b) geht man im Allgemeinen wie folgt vor. Die Verbindungen der Formel (III) werden gegebenenfalls in Gegenwart eines Verdünnungsmittels mit einer Base und 1,4-Diazabicyclo[2.2.2]-octan versetzt. Hierzu gibt man die Verbindungen der Formel (VI) gegebenenfalls in Gegenwart eines Verdünnungsmittels und rührt, gegebenenfalls bei erhöhter Temperatur. Nach beendeter Reaktion wird das Reaktionsgemisch in üblicher Weise aufgearbeitet.

Alternativ kann die Durchführung des Verfahrensschritts b) auch dadurch erfolgen, dass die Verbindungen der Formel (VI) gegebenenfalls in Gegenwart eines Verdünnungsmittels mit einer Base und 1,4-Diazabicyclo[2.2.2]octan versetzt werden. Hierzu gibt man die Verbindungen der Formel (III) gegebenenfalls in Gegenwart eines Verdünnungsmittels und rührt, gegebenenfalls bei erhöhter Temperatur. Nach beendeter Reaktion wird das Reaktionsgemisch in üblicher Weise aufgearbeitet. Eine spezielle Variante des erfindungsgemäßen Verfahrens ist die Durchführung als Eintopfreaktion.

Die nachfolgenden Beispiele dienen der Erläuterung der Erfindung. Die Erfindung ist jedoch nicht auf die Beispiele limitiert.

### Beispiele

### Beispiel 1

### Verfahrensvariante a) oder b)

### 4-Chlor-5-fluor-6-[4-fluor-3-(trifluormethyl)phenoxy]pyrimidin

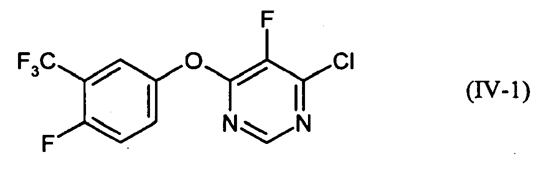

### (erste Stufe)

4,6-Dichlor-5-fluorpyrimidin (1,67 g, Gehalt: 98,9 %) wird mit Kaliumcarbonat (1,72 g) in Methylisobutylketon (5 ml) vorgelegt und bei 60°C über einen Zeitraum von 3 Stunden mit einer Lösung von 1,8 g 4-Fluor-3-(trifluormethyl)phenol in 5 ml Methylisobutylketon tropfenweise versetzt. Man rührt 1,5 Stunden bei 60°C, versetzt nach Abkühlung mit Wasser, trennt die organische Phase ab, extrahiert die wässrige Phase erneut mit Methylisobutylketon, vereinigt die organischen Extrakte, trocknet über Natriumsulfat und destilliert das Lösungsmittel ab. Man erhält 4-Chlor-5-fluor-6-[4-fluor-3-(trifluormethyl)phenoxy]pyrimidin (2,74 g, Gehalt: 93,9%, 83,8% d.Th.) als Öl.

### 4-[4-Chlor-3-(trifluormethyl)phenoxy]5-fluor-6-[4-fluor-3-(trifluormethyl)-phenoxy]pyrimidin

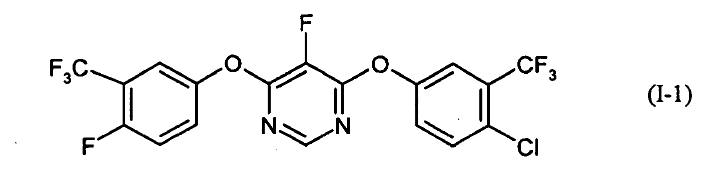

### (zweite Stufe)

4-Chlor-3-(trifluormethyl)phenol (0,98 g) wird mit Kaliumcarbonat (0,9 g) und 1,4-Diazabicyclooctan[2.2.2] (DABCO) (28 mg) in einem Methylisobutylketon-Wasser-Gemisch (8 ml, 7/1) vorgelegt und bei 70-80°C mit einer Lösung von 4-Chlor-5-fluor-6-[4-fluor-3-(trifluormethyl)phenoxy]pyrimidin (1,56 g, Gehalt: 98,8 %) in 7 ml Methylisobutylketon versetzt. Man rührt 2 Stunden bei 70-80°C, gibt nach Abkühlung Wasser hinzu, trennt die organische Phase ab, extrahiert die wässrige Phase mit Methylisobutylketon, trocknet die vereinigten organischen Extrakte über Natriumsulfat und destilliert das Lösungsmittel ab. Man erhält 4-[4-Chlor-3-trifluormethyl)phenoxy]-5-fluor-6-[4-fluor-3-(trifluormethyl)phenoxy]pyrimidin (1,92 g, Gehalt: 96,5 %, 79,2 % d. Th.) als Feststoff.

### Beispiel 2

### Verfahrensvariante a)

### 4-Chlor-6-(3-chlor-2-methylphenoxy)-5-fluorpyrimidin

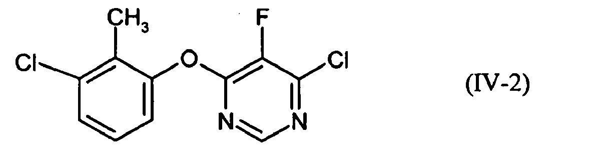

### (erste Stufe)

4,6-Dichlor-5-fluorpyrimidin (16,7 g, Gehalt: 99,7 %) wird mit Kaliumcarbonat (20,2 g) in Methylisobutylketon (50 ml) vorgelegt und bei 60°C über einen Zeitraum von 3,5 Stunden mit einer Lösung von 14,3 g 3-Chlor-2-methylphenol in 50 ml Methylisobutylketon tropfenweise versetzt. Man rührt 2 Stunden bei 60°C, versetzt nach Abkühlung mit Wasser, trennt die organische Phase ab, extrahiert die wässrige Phase erneut mit Methylisobutylketon, vereinigt die organischen Extrakte, trocknet über Natriumsulfat und destilliert das Lösungsmittel ab. Man erhält 4-Chlor-6-(3-chlor-2-methylphenoxy)-5-fluorpyrimidin (26,8 g, Gehalt: 96,7 %, 95,2 % d.Th.) als Feststoff.

### (2E)-2-(2-{[6-(3-Chlor-2-methylphenoxy)-5-fluor-4-pyrimidinyl]oxy}phenyl)-2-(methoxyimino)-N-methylethanamid

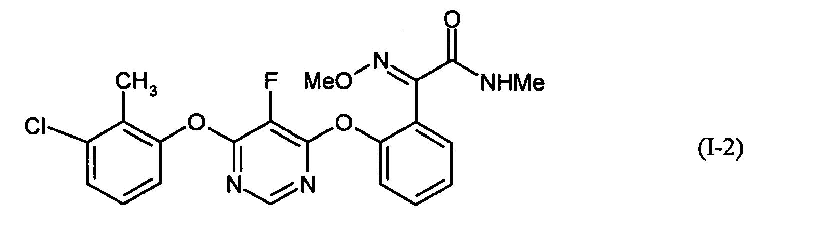

### (zweite Stufe)

(2E)-2-(2-Hydroxyphenyl)-2-(methoxyimino)-N-methylethanamid (4,16 g, Gehalt: 99,7 %) wird mit Kaliumcarbonat (3,7 g) und DABCO (110 mg) in Methylisobutylketon (30 ml) vorgelegt und bei 50°C mit einer Lösung von 4-Chlor-6-(3-chlor-2-methylphenoxy)-5-fluorpyrimidin (5,46 g, Gehalt. 98.1 %) in 30 ml Methylisobutylketon versetzt. Man rührt 4 Stunden bei 50°C, gibt nach Abkühlung Wasser hinzu, trennt die organische Phase ab, extrahiert die wässrige Phase mit Methylisobutylketon, trocknet die vereinigten die organischen Extrakte über Natriumsulfat und destilliert das Lösungsmittel ab. Man erhält (2E)-2-(2-{[6-(3-Chlor-2-methyl-phenoxy)-5-fluor-4-pyrimidinyl]oxy}phenyl)-2-(methoxyimino)-N-methylethanamid (9,15 g, Gehalt: 94,2 %, 96,9 % d.Th.) als Öl.

### Beispiel 3

### Verfahrensvariante a)

### 4-Chlor-6-(2-chlorphenoxy)-5-fluorpyrimidin

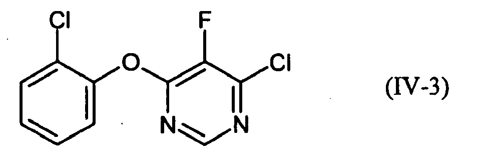

### (erste Stufe)

4,6-Dichlor-5-fluorpyrimidin (33,5 g, Gehalt: 98,9 %) wird mit Kaliumcarbonat (34,4 g) in einem Methylisobutylketon-Wasser-Gemisch (120 ml, 5/1) vorgelegt und bei 60°C über einen Zeitraum von 3 Stunden mit einer Lösung von 25,7 g o-Chlorphenol in 100 ml Methylisobutylketon tropfenweise versetzt. Man rührt 6 Stunden bei 60°C, trennt nach Abkühlung die organische Phase ab, wäscht die organische Phase mit 5 % NaOH, extrahiert die wässrige Phase mit Methylisobutylketon, vereinigt die organischen Extrakte, trocknet über Natriumsulfat und destilliert das Lösungsmittel ab. Man erhält 4-Chlor -6-(2-chlorphenoxy)-5-fluorpyrimidin (48,4 g, Gehalt: 95,6 %, 90,3 % d.Th.) als Öl.

### (E)-(2-{[6-(2-Chlorphenoxy)-5-fluor-4-pyrimidinyl]oxy}phenyl)-(5,6-dihydro-1,4,2-dioxazin-3-yl)methanon O-methyloxim

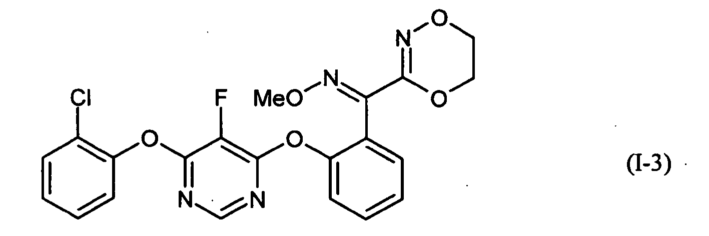

### (zweite Stufe)

(E)-5,6-Dihydro-1,4,2-dioxazin-3-yl(2-hydroxyphenyl)methanon O-methyloxim (11,8 g) wird mit Kaliumcarbonat (9,0 g) und DABCO (280 mg) in einem Methylisobutylketon-Wasser-Gemisch (80 ml, 7/1) vorgelegt und bei 80°C mit einer Lösung von 4-Chlor-6-(2-chlorphenoxy)-5-fluorpyrimidin (13,1 g, Gehalt. 98,1 %) in 70 ml Methylisobutylketon versetzt. Man rührt 1,5 Stunden bei 80°C, gibt nach Abkühlung Wasser hinzu, trennt die organische Phase ab, extrahiert die wässrige Phase mit Methylisobutylketon, trocknet die vereinigten organischen Extrakte über Natriumsulfat und destilliert das Lösungsmittel ab. Man erhält (E)-2-(2-{[6-(2-Chlorphenoxy)-5-fluor-4-pyrimidinyl]oxy}phenyl)(5,6-dihydro-1,4,2-dioxazin-3-yl)-methanon O-methyloxim (23,4 g, Gehalt: 95,2 %, 97,9 % d.Th.) als Feststoff.

### Beispiel 4

### Verfahrensvariante a)

### 4-Chlor-6-(2-chlorphenoxy)-5-fluorpyrimidin

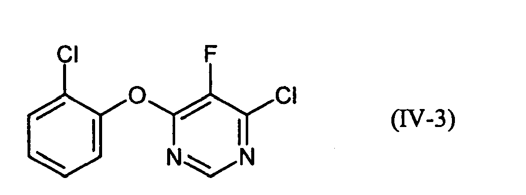

### (erste Stufe)

4,6-Dichlor-5-fluorpyrimidin (33,5 g, Gehalt: 98,9 %) wird mit Kaliumcarbonat (34,4 g) in einem Methylisobutylketon-Wasser-Gemisch (120 ml, 5/1) vorgelegt und bei 60°C über einen Zeitraum von 3 Stunden mit einer Lösung von 25,7 g o-Chlorphenol in 100 ml Methylisobutylketon tropfenweise versetzt. Man rührt 6 Stunden bei 60°C, trennt nach Abkühlung die organische Phase ab, wäscht die organische Phase mit 5 % NaOH, extrahiert die wässrige Phase mit Methylisobutylketon, vereinigt die organischen Extrakte, trocknet über Natriumsulfat und destilliert das Lösungsmittel ab. Man erhält 4-Chlor -6-(2-chlorphenoxy)-5-fluorpyrimidin (48,4 g, Gehalt: 95,6 %, 90,3 % d.Th.) als Öl.

### (E)-(2-{[6-(2-Chlorphenoxy)-5-fluor-4-pyrimidinyl]oxy}phenyl)-(5,6-dihydro-1,4,2-diozazin-3-yl)methanon O-methyloxim

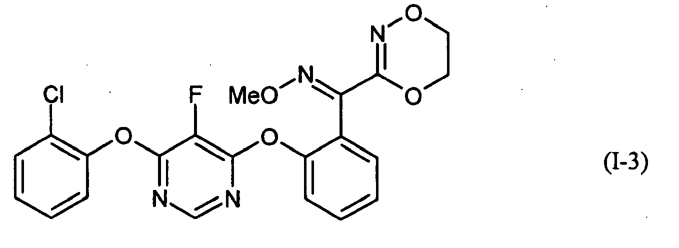

### (zweite Stufe, Vergleichsversuch)

(E)-5,6-Dihydro-1,4,2-dioxazin-3-yl(2-hydroxyphenyl)methanon O-methyloxim (11,8 g) wird mit Kaliumcarbonat (9,0 g) in einem Methylisobutylketon-Wasser-Gemisch (80 ml, 7/1) vorgelegt und bei 50°C mit einer Lösung von 4-Chlor-6-(2-chlorphenoxy)-5-fluorpyrimidin (13,1 g, Gehalt. 98,1 %) in 70 ml Methylisobutylketon versetzt. Man rührt 24 Stunden bei 50°C, gibt nach Abkühlung Wasser hinzu, trennt die organische Phase ab, extrahiert die wässrige Phase mit Methylisobutylketon, trocknet die vereinigten organischen Extrakte über Natriumsulfat und destilliert das Lösungsmittel ab. Man erhält (E)-2-(2-{[6-(2-Chlorphenoxy)-5-fluor-4-pyrimidinyl]oxy}phenyl)(5,6-dihydro-1,4,2-dioxazin-3-yl)methanon O-methyloxim (26,4 g, Gehalt: 33,3 %, 38,6 % d.Th.) als Öl.

### Beispiel 5

### Verfahrensvariante b)

### (E)-{2-[(6-Chlor-5-fluor-4-pyrimidinyl)oxy]phenyl}(5,6-dihydro-1,4,2,-dioxazin-3-yl)methanon O-methyloxim

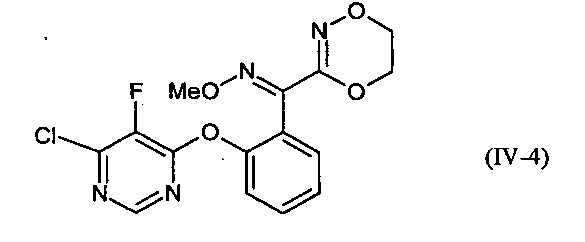

### (erste Stufe)

4,6-Dichlor-5-fluorpyrimidin (31,8 g, Gehalt: 98,9 %) wird mit Kaliumcarbonat (31,5 g) in Aceton (115 ml) vorgelegt und bei 60°C über einen Zeitraum von 6 Stunden mit einer Lösung von 44,9 g (E)-5,6-Dihydro-1,4,2-dioxazin-3-yl(2-hydroxyphenyl)methanon O-methyloxim in 350 ml Aceton tropfenweise versetzt. Man rührt 2 Stunden bei 60°C, destilliert das Aceton ab, versetzt mit Methylenchlorid und Wasser, trennt die organische Phase ab, extrahiert die wässrige Phase mit Methylenchlorid, vereinigt die organischen Extrakte, wäscht diese mit 5 % NaOH, trocknet über Natriumsulfat und destilliert das Lösungsmittel ab. Man erhält (E)-{2-[(6-Chlor-5-fluor-4-pyrimidinyl)oxy]phenyl}(5,6-dihydro-1,4,2,-dioxazin-3-yl)-methanon O-methyloxim (68,0 g, Gehalt: 95,8 %, 94,5 % d.Th.) als Feststoff.

## Patentansprüche

1. Verfahren zur Herstellung von Verbindungen der allgemeinen Formel (I), 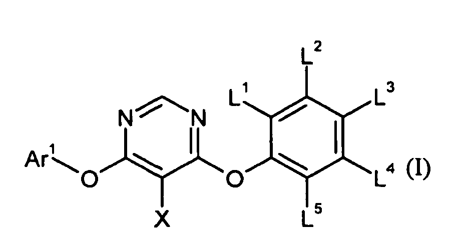 in welcher
Ar¹ für gegebenenfalls durch Halogen oder durch Alkyl, Alkoxy, Halogenalkyl, Halogenalkoxy mit jeweils 1 bis 4 Kohlenstoffatomen substituiertes Heterocyclyl mit 3 bis 7 Ringgliedern steht;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Formyl, Carboxy, Carbamoyl, Thiocarbamoyl;
jeweils geradkettiges oder verzweigtes Alkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Dialkylamino,
Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl, mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung 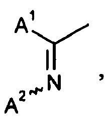 worin
A¹ für Wasserstoff, Hydroxy oder Alkyl mit 1 bis 4 Kohlenstoffatomen oder Cycloalkyl mit 1 bis 6 Kohlenstoffatomen steht und
A² für Hydroxy, Methoxy, Ethoxy, Amino, Methylamino, Phenyl, Benzyl oder für jeweils gegebenenfalls durch Cyano, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht,
sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Benzoyl, Benzoylethenyl, Cinnamoyl, Heterocyclyl oder Phenylalkyl, Phenylalkyloxy oder Heterocyclylalkyl, mit jeweils 1 bis 3 Kohlenstoffatomen in den jeweiligen Alkylteilen,
X für Fluor oder Chlor steht,
L¹, L², L³, L⁴ und L⁵ gleich oder verschieden sind und unabhängig voneinander jeweils für Wasserstoff, Halogen, Cyano, Nitro, Formyl, Alkylcarbonyl, Alkoxycarbonyl, Aminocarbonyl, Alkylaminocarbonyl, Dialkylaminocarbonyl mit jeweils 1 bis 6 Kohlenstoffatomen, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen stehen,
oder
L¹, L², L³ und L⁴ gleich oder verschieden sind und unabhängig voneinander jeweils für Wasserstoff, Halogen, Cyano, Nitro, Formyl, Alkylcarbonyl, Alkoxycarbonyl, Aminocarbonyl, Alkylaminocarbonyl, Dialkylaminocarbonyl, mit jeweils 1 bis 6 Kohlenstoffatomen, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen stehen und
und
L⁵ für eine der folgenden Gruppen steht: 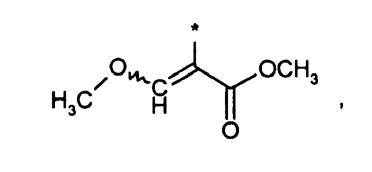 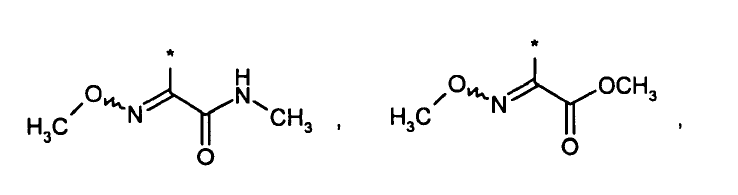 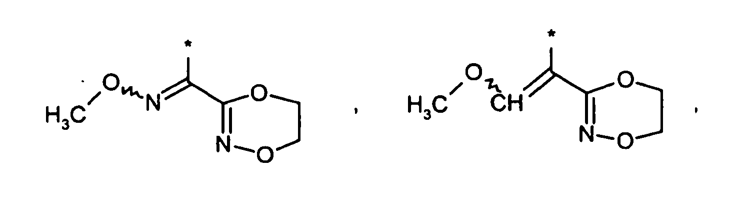 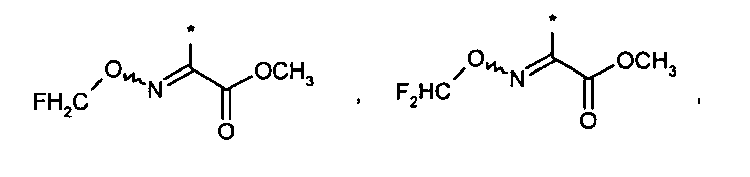 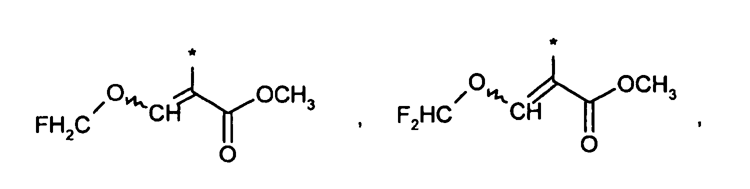 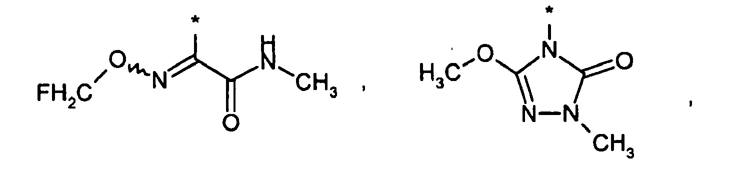
wobei * die Anknüpfungsstelle an den Phenylrest bezeichnet,
und wobei die Reste
Ar¹ und 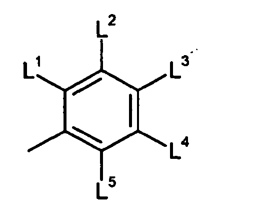 verschieden sind,
**dadurch gekennzeichnet, dass** man
4,6-Dichlorpyrimidin-Derivate der allgemeinen Formel (II), 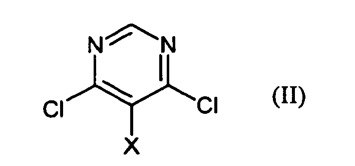 in welcher
X die oben angegebene Bedeutung hat,
a) zunächst in einer ersten Stufe mit Verbindungen der allgemeinen Formel (III),
Ar¹-OH, (III)
in welcher
Ar¹ die oben angegebene Bedeutung hat,
gegebenenfalls in Gegenwart eines Verdünnungsmittels und gegebenenfalls in Gegenwart eines Säureakzeptors umsetzt,
und die dabei entstehenden Verbindungen der Formel (IV), 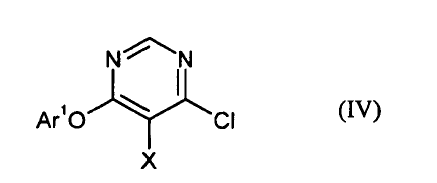 in welcher
Ar¹ und X die oben angegebenen Bedeutungen haben,
dann in einer zweiten Stufe mit Verbindungen der allgemeinen Formel (V), 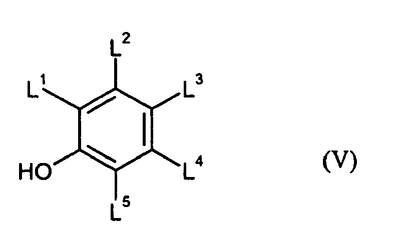 in welcher
L¹, L², L³, L⁴ und L⁵ die oben angegebenen Bedeutungen haben,
gegebenenfalls in Gegenwart eines Lösungsmittel, in Gegenwart einer Base und unter Zusatz von 2 bis 20 Mol-% 1,4-Diazabicyclo[2.2.2]octan (DABCO) umsetzt,
oder
b) zunächst in einer ersten Stufe mit Verbindungen der allgemeinen Formel (V), 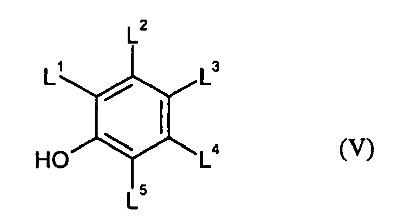 in welcher
L¹, L², L³, L⁴ und L⁵ die oben angegebenen Bedeutungen haben,
gegebenenfalls in Gegenwart eines Verdünnungsmittels und gegebenenfalls in Gegenwart eines Säureakzeptors umsetzt,
und die dabei entstehenden Verbindungen der Formel (VI), 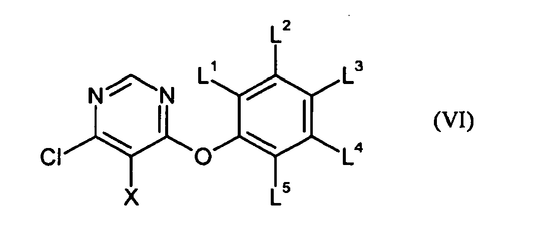 in welcher
X, L¹, L², L³, L⁴ und L⁵ die oben angegebenen Bedeutungen haben,
dann in einer zweiten Stufe mit Verbindungen der allgemeinen Formel (III),
Ar¹-OH, (III)
in welcher
Ar¹ die oben angegebene Bedeutung hat,
gegebenenfalls in Gegenwart eines Lösungsmittel, in Gegenwart einer Base und unter Zusatz von 2 bis 20 Mol-% 1,4-Diazabicyclo[2.2.2]octan (DABCO) umsetzt.

2. Verfahren gemäß Anspruch 1, **dadurch gekennzeichnet, dass** das Verfahren zur Herstellung der Verbindungen der Formel (I) als Eintopfverfahren durchgeführt wird.

3. Verfahren gemäß Anspruch 1 oder 2, **dadurch gekennzeichnet, dass** pro Mol der Verbindungen der Formel (III) 1 bis 4 Mol des 4,6-Dichlorpyrimidin-Derivats der Formel (II) eingesetzt werden.

4. Verfahren gemäß Anspruch 1 oder 2, **dadurch gekennzeichnet, dass** pro Mol der Verbindungen der Formel (V) 1 bis 4 Mol des 4,6-Dichlorpyrimidin-Derivats der Formel (II) eingesetzt werden.

5. Verfahren gemäß mindestens einem der Ansprüche 1 bis 4, **dadurch gekennzeichnet, dass** pro Mol der Verbindungen der Formel (IV) 0,8 bis 4 Mol der Verbindungen der Formel (V) eingesetzt werden.

6. Verfahren gemäß mindestens einem der Ansprüche 1 bis 4, **dadurch gekennzeichnet, dass** pro Mol der Verbindungen der Formel (VI) 0,8 bis 4 Mol der Verbindungen der Formel (III) eingesetzt werden.

7. Verfahren gemäß mindestens einem der Ansprüche 1 bis 6, **dadurch gekennzeichnet, dass**
Ar¹ für gegebenenfalls durch Methyl, Ethyl, Methoxy, Ethoxy, Trifluormethyl oder Trifluormethoxy substituiertes Thienyl, Pyridyl oder Furyl steht;
oder für jeweils gegebenenfalls einfach bis vierfach, gleich oder verschieden substituiertes Phenyl steht, wobei die möglichen Substituenten vorzugsweise aus der nachstehenden Aufzählung ausgewählt sind:
Fluor, Chlor, Brom, Iod, Cyano, Nitro, Formyl, Carboxy, Carbamoyl, Thiocarbamoyl, Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), 3-Oxobutyl, Methoxymethyl, Dimethoxymethyl,
Methoxy, Ethoxy, n- oder i-Propoxy,
Methylthio, Ethylthio, n- oder i-Propylthio, Methylsulfinyl, Ethylsulfinyl, Methylsulfonyl oder Ethylsulfonyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy,
oder eine Gruppierung 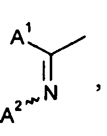 wobei
A¹ für Wasserstoff, Methyl oder Hydroxy steht und
A² für Hydroxy, Methoxy, Ethoxy, Amino, Methylamino, Phenyl oder Benzyl steht, sowie
jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Benzoyl, Benzoylethenyl, Cinnamoyl, Benzyl, Phenylethyl, Phenylpropyl, Benzyloxy, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazolyl.
X für Fluor steht,
L¹, L², L³ und L⁴ gleich oder verschieden sind und unabhängig voneinander jeweils für Wasserstoff, Fluor, Chlor, Brom, Cyano, Nitro, Acetyl, Propionyl, Methoxycarbonyl, Ethoxycarbonyl, Methylaminocarbonyl, Ethylaminocarbonyl, Dimethylaminocarbonyl, Diethylaminocarbonyl, Methyl, Ethyl, n- oder i-Propyl, n-, i-, s- oder t-Butyl, Methoxy, Ethoxy, n- oder i-Propoxy, Methylthio, Ethylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl stehen, und
L⁵ für eine der folgenden Gruppen steht: 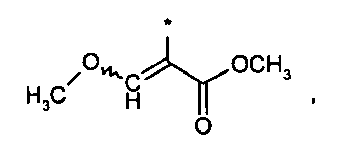 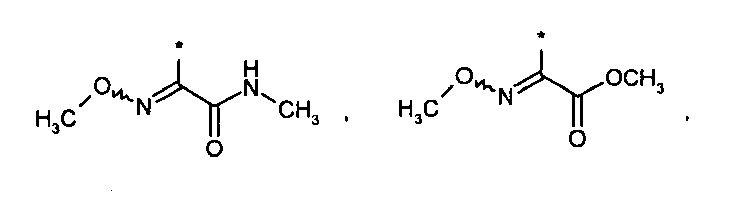 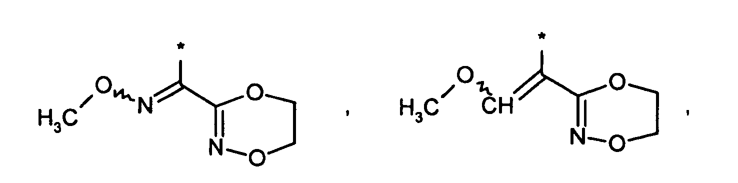 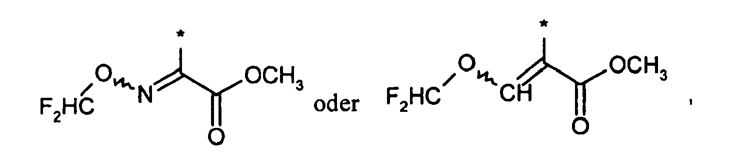
wobei * die Anknüpfungsstelle an den Phenylrest bezeichnet.

8. Verfahren gemäß mindestens einem der Ansprüche 1 bis 7, **dadurch gekennzeichnet, dass**
Ar¹ für gegebenenfalls durch Methyl, Ethyl, Methoxy, Ethoxy, Trifluormethyl oder Trifluormethoxy substituiertes Thienyl, Pyridyl oder Furyl steht;
oder für jeweils gegebenenfalls einfach bis vierfach, gleich oder verschieden substituiertes Phenyl steht, wobei die möglichen Substituenten vorzugsweise aus der nachstehenden Aufzählung ausgewählt sind:
Fluor, Chlor, Brom, Iod, Cyano, Nitro, Formyl, Carboxy, Carbamoyl, Thiocarbamoyl,
Methyl, Ethyl, n- oder i-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-, 2-, 3-(3-Methylpentyl), 2-Ethylbutyl, 1-, 3-, 4-(2,2-Dimetylbutyl), 1-, 2-(2,3-Dimethylbutyl), 3-Oxobutyl, Methoxymethyl, Dimethoxymethyl,
Methoxy, Ethoxy, n- oder i-Propoxy,
Methylthio, Ethylthio, n- oder i-Propylthio, Methylsulfinyl, Ethylsulfinyl, Methylsulfonyl oder Ethylsulfonyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Dimethylamino, Diethylamino,
Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy,
oder eine Gruppierung 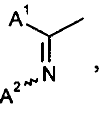 wobei
A¹ für Wasserstoff, Methyl oder Hydroxy steht und
A² für Hydroxy, Methoxy, Ethoxy, Amino, Methylamino, Phenyl oder Benzyl steht, sowie
jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Benzyl, Phenylethyl, Phenylpropyl, Benzyloxy, Benzylthio, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazolyl,
X für Fluor steht,
L¹, L², L³ und L⁴ für Wasserstoff stehen,
und
L⁵ für eine der folgenden Gruppen steht: 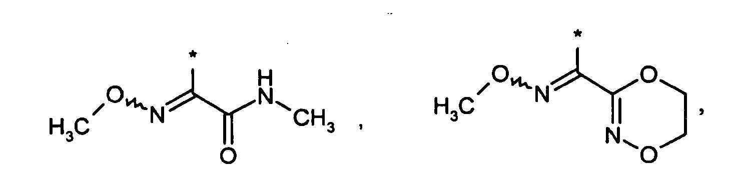
wobei * die Anknüpfungsstelle an den Phenylrest bezeichnet.

9. Verfahren gemäß mindestens einem der Ansprüche 1 bis 8, **dadurch gekennzeichnet, dass** man die Verbindung der Formel (I-3) als Reaktionsprodukt 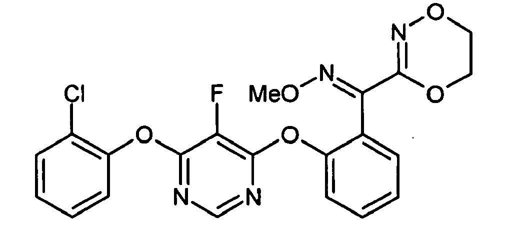 erhält.

10. Verfahren gemäß mindestens einem der Ansprüche 1 bis 9, **dadurch gekennzeichnet, dass** man als Base in der ersten Stufe Kalium- oder Natriumcarbonat verwendet.

11. Verfahren gemäß mindestens einem der Ansprüche 1 bis 10, **dadurch gekennzeichnet, dass** man als Lösungsmittel in der ersten Stufe Methylisobutylketon verwendet.

12. Verfahren gemäß mindestens einem der Ansprüche 1 bis 11, **dadurch gekennzeichnet, dass** man in der ersten Stufe bei Temperaturen von 40 - 80°C arbeitet.

13. Verfahren gemäß mindestens einem der Ansprüche 1 bis 12, **dadurch gekennzeichnet, dass** man als Base in der zweiten Stufe Kalium- oder Natriumcarbonat verwendet.

14. Verfahren gemäß mindestens einem der Ansprüche 1 bis 13, **dadurch gekennzeichnet, dass** man als Lösungsmittel in der zweiten Stufe Methylisobutylketon verwendet.

15. Verfahren gemäß mindestens einem der Ansprüche 1 bis 14, **dadurch gekennzeichnet, dass** man in der zweiten Stufe bei Temperaturen von 50 - 80°C arbeitet.

## Claims

1. Process for preparing compounds of the general formula (I), 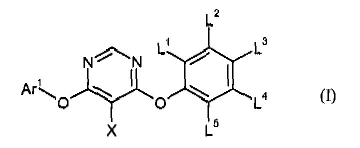 in which
Ar¹ represents heterocyclyl having 3 to 7 ring members which is optionally substituted by halogen or by alkyl, alkoxy, halogenoalkyl, halogenoalkoxy having in each case 1 to 4 carbon atoms;
or represents phenyl or naphthyl, each of which is optionally mono- to tetrasubstituted by identical or different substituents, the possible substituents preferably being selected from the list bellow:
halogen, cyano, nitro, formyl, carboxyl, carbamoyl, thiocarbamoyl;
in each case straight-chain or branched alkyl, oxoalkyl, alkoxy, alkoxyalkyl, alkylthioalkyl, dialkoxyalkyl, alkylthio, alkylsulphinyl or alkylsulphonyl having in each case 1 to 8 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and to 13 identical or different halogen atoms;
in each case straight-chain or branched halogenoalkenyl or halogenoalkenyloxy having in each case 2 to 6 carbon atoms and 1 to 11 identical or different halogen atoms;
in each case straight-chain or branched Dialkylamino,
alkylcarbonyl, alkylcarbonyloxy, alkoxycarbonyl, alkylaminocarbonyl, dialkylaminocarbonyl, arylalkylaminocarbonyl, dialkylaminocarbonyloxy, alkenylcarbonyl or alkinylcarbonyl, having 1 to 6 carbon atoms in the respective hydrocarbon chain;
cycloalkyl or cycloalkyloxy having in each case from 3 to 6 carbon atoms;
in each case doubly attached alkylene having 3 or 4 carbon atoms, oxyalkylene having 2 or 3 carbon atoms or dioxyalkylene having 1 or 2 carbon atoms, each of which is optionally mono- to tetrasubstituted by identical or different substituents from the group consisting of fluorine, chlorine, oxo, methyl, trifluoromethyl and ethyl;
or a grouping 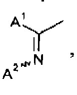 in which
A¹ represents hydrogen, hydroxyl or alkyl having 1 to 4 carbon atoms or cycloalkyl having 1 to 6 carbon atoms and
A² represents hydroxyl, methoxy, ethoxy, amino, methylamino, phenyl, benzyl or represents in each case optionally cyano-, alkoxy-, alkylthio-, alkylamino-, dialkylamino- or phenyl-substituted alkyl or alkoxy having 1 to 4 carbon atoms, or represents alkenyloxy or alkinyloxy having in each case 2 to 4 carbon atoms,
and also phenyl, benzoyl, benzoylethenyl, cinnamoyl, heterocyclyl or phenylalkyl, phenylalkyloxy or heterocyclylalkyl, having in each case I to 3 carbon atoms in the respective alkyl moieties and being in each case optionally mono- to trisubstituted in the ring moiety by halogen and/or straight-chain or branched alkyl or alkoxy having I to 4 carbon atoms,
X represents fluorine or chlorine,
L¹, L², L³, L⁴ and L⁵ are identical or different and independently of one another each represents hydrogen, halogen, cyano, nitro, formyl, alkylcarbonyl, alkoxycarbonyl, aminocarbonyl, alkylaminocarbonyl, dialkylaminocarbonyl having in each case I to 6 carbon atoms, alkyl, alkoxy, alkylthio, alkylsulphinyl or alkylsulphonyl having in each case 1 to 6 carbon atoms and being in each case optionally substituted by 1 to 5 halogen atoms,
or
L¹, L², L³ and L⁴ are identical or different and independently of one another each represents hydrogen, halogen, cyano, nitro, formyl, alkylcarbonyl, alkoxycarbonyl, aminocarbonyl, alkylaminocarbonyl, dialkylaminocarbonyl having in each case 1 to 6 carbon atoms, alkyl, alkoxy, alkylthio, alkylsulphinyl or alkylsulphonyl having in each case 1 to 6 carbon atoms and being in each case optionally substituted by 1 to 5 halogen atoms and
L⁵ represents one of the groups bellow: 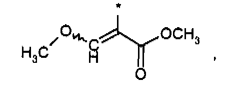 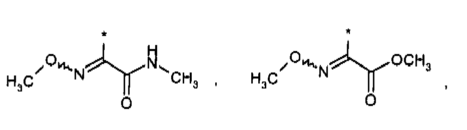 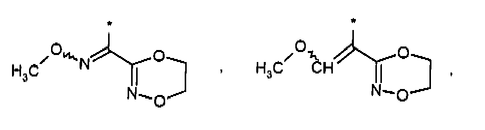 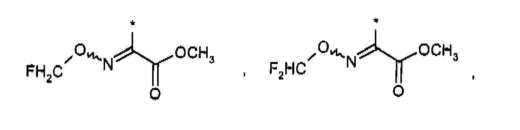 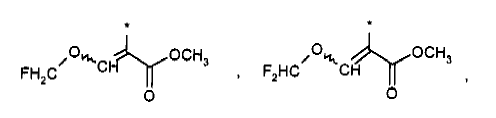 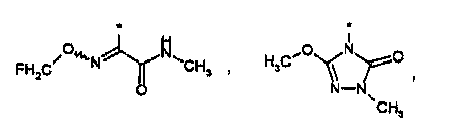 where * denotes the point of attachment to the phenyl radical,
and where the radicals 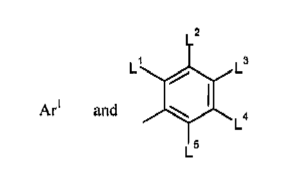 are different,
**characterized in that**
4,6-dichloropyrimidine derivatives of the general formula (II), 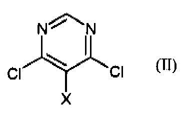 in which
X is as defined above,
a) are initially, in a first step, reacted with compounds of the general formula (III),
Ar¹-OH, (III)
in which
Ar¹ is as defined above,
if appropriate in the presence of a diluent and if appropriate in the presence of an acid acceptor,
and the resulting compounds of formula (IV), 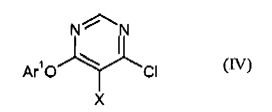 in which
Ar¹ and X are each as defined above
are then, in a second step, reacted with compounds of the general formula (V), 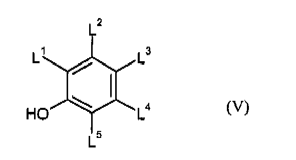 in which
L¹, L², L³, L⁴ and L⁵ are each as defined above,
if appropriate in the presence of a solvent, in the presence of a base and with addition of from 2 to 40 mol% of 1,4-diazabicyclo[2.2.2]octane (DABCO),
or
b) are initially, in a first step, reacted with compounds of the general formula (V), 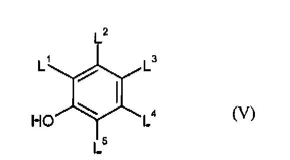 in which
L¹, L², L³, L⁴ and L⁵ are each as defined above,
if appropriate in the presence of a diluent and if appropriate in the presence of an acid acceptor,
and the resulting compounds of the formula (VI), 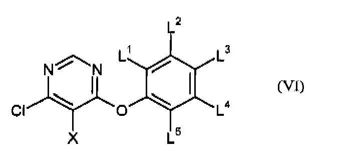 in which
X, L¹, L², L³, L⁴ and L⁵ are each as defined above,
are then, in a second step, reacted with compounds of general formula (III),
Ar¹-OH, (III)
in which
Ar¹ is as defined above,
if appropriate in the presence of a solvent, in the presence of a base and with addition of from 2 to 40 mol% of 1,4-diazabicyclo[2.2.2]octane (DABCO).

2. Process according to Claim 1, **characterized in that** the process for preparing the compounds of the formula (I) is carried out as a one-pot process.

3. Process according to Claim 1 or 2, **characterized in that** from I to 4 mol of the 4,6-dichloropyrimidine derivative of the formula (II) are employed per mole of the compounds of the formula (III).

4. Process according to Claim 1 or 2, **characterized in that** from 1 to 4 mol of the 4,6-dichloropyrimidine derivative of the formula (II) are employed per mole of the compounds of the formula (V).

5. Process according to at least one of Claims 1 to 4, **characterized in that** from 0.8 to 4 mol of the compounds of formula (V) are employed per mole of the compounds of the formula (IV).

6. Process according to at least one of Claims 1 to 4, **characterized in that** from 0.8 to 4 mol of the compounds of formula (III) are employed per mole of the compounds of the formula (VI).

7. Process according to at least one of Claims 1 to 6, **characterized in that**
Ar¹ represents optionally methyl-, ethyl-, methoxy-, ethoxy-, trifluoromethyl- or trifluoromethoxy-substituted thienyl, pyridyl or furyl;
or represents phenyl which is in each case optionally mono- to tetrasubstituted by identical or different substituents, the possible substituents preferably being selected from the list below:
fluorine, chlorine, bromine, iodine, cyano, nitro, formyl, carboxyl, carbamoyl, thiocarbamoyl,
methyl, ethyl, n- or i-propyl, n-, i-, s- or t-butyl, 1-, 2-, 3-, neo-pentyl, 1-, 2-, 3-, 4-(2-methylbutyl), 1-, 2-, 3-hexyl, 1-, 2-, 3-, 4-, 5-(2-methylpentyl), 1-, 2-, 3-(3-methylpentyl), 2-ethylbutyl, 1-, 3-, 4-(2,2-dimetylbutyl), 1-, 2-(2,3-dimethylbutyl), 3-oxobutyl, methoxymethyl, dimethoxymethyl,
methoxy, ethoxy, n- or i-propoxy,
methylthio, ethylthio, n- oder i-propylthio, methylsulphinyl, ethylsulphinyl, methylsulphonyl or ethylsulphonyl,
vinyl, allyl, 2-methylallyl, propene-1-yl, crotonyl, propargyl, vinyloxy, allyloxy, 2-methylallyloxy, propene-1-yloxy, crotonyloxy, propargyloxy;
trifluoromethyl, trifluoroethyl,
difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl,
dimethylamino, diethylamino,
acetyl, propionyl, methoxycarbonyl, ethoxycarbonyl, methylaminocarbonyl, ethylaminocarbonyl, dimethylaminocarbonyl, diethylaminocarbonyl, dimethylaminocarbonyloxy, diethylaminocarbonyloxy, benzylaminocarbonyl, acryloyl, propioloyl,
cyclopentyl, cyclohexyl,
in each case doubly attached propanediyl, ethyleneoxy, each of which is optionally mono- to tetrasubstituted by identical or different substituents from the group consisting of fluorine, chlorine, oxo, methyl and trifluoromethyl,
or a grouping 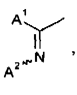 where
A¹ represents hydrogen, methyl or hydroxyl and
A² represents hydroxyl, methoxy, ethoxy, amino, methylamino, phenyl or benzyl, and also
phenyl, benzoyl, benzoylethenyl, cinnamoyl, benzyl, phenylethyl, phenylpropyl, benzyloxy, 5,6-dihydro-1,4,2-dioxazin-3-ylmethyl, triazolylmethyl, benzoxazol-2-ylmethyl, 1,3-dioxan-2-yl, benzimidazol-2-yl, dioxol-2-yl, oxidiazolyl, each of which is optionally mono- to trisubstituted in the ring moiety by halogen and/or straight-chain or branched alkyl or alkoxy having 1 to 4 carbon atoms.
X represents fluorine,
L¹, L², L³ and L⁴ are identical or different and independently of one another each represents hydrogen, fluorine, chlorine, bromine, cyano, nitro, acetyl, propionyl, methoxycarbonyl, ethoxycarbonyl, methylaminocarbonyl, ethylaminocarbonyl, dimethylaminocarbonyl, diethylaminocarbonyl, methyl, ethyl, n- or i-propyl, n-, i-, s- or t-butyl, methoxy, ethoxy, n- or i-propoxy, methylthio, ethylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, and
L⁵ represents one of the groups below: 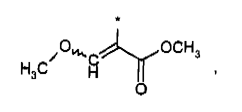 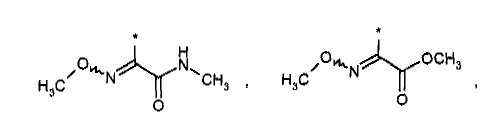 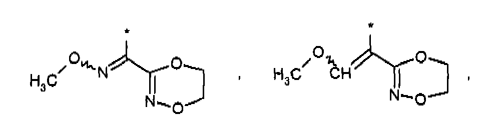 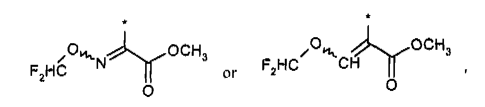 where * denotes the point of attachment to the phenyl radical.

8. Process according to at least one of Claims 1 to 7, **characterized in that**
Ar¹ represents optionally methyl-, ethyl-, methoxy-, ethoxy-, trifluoromethyl- or trifluoromethoxy-substituted thienyl, pyridyl or furyl;
or represents phenyl which is in each case optionally mono- to tetrasubstituted by identical or different substituents, the possible substituents preferably being selected from the list below:
fluorine, chlorine, bromine, iodine, cyano, nitro, formyl, carboxyl, carbamoyl, thiocarbamoyl,
methyl, ethyl, n- or i-propyl, n-, i-, s- or t-butyl, 1-, 2-, 3-, neo-pentyl, 1-, 2-, 3-, 4-(2-methylbutyl), 1-, 2-, 3-hexyl, 1 2-, 3-, 4-, 5-(2-methylpentyl), 1 2-, 3-(3-methylpentyl), 2-ethylbutyl, 1-, 3-, 4-(2,2-dimetylbutyl), 1-, 2-(2,3-dimethylbutyl), 3-oxobulyl, methoxymethyl, dimethoxymethyl,
methoxy, ethoxy, n- or i-propoxy,
methylthio, ethylthio, n - oder i-propylthio, methylsulphinyl, ethylsulphinyl, methylsulphonyl or ethylsulphonyl,
vinyl, allyl, 2-methylallyl, propene-1-yl, crotonyl, propargyl, vinyloxy, allyloxy, 2-methylallyloxy, propene-1-yloxy, crotonyloxy, propargyloxy;
trifuoromethyl, trifluoroethyl,
difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl,
dimethylamino, diethylamino,
acetyl, propionyl, methoxycarbonyl, ethoxycarbonyl, methylaminocarbonyl, ethylaminocarbonyl, dimethylaminocarbonyl, diethylaminocarbonyl, dimethylaminocarbonyloxy, diethylaminocarbonyloxy, benzylaminocarbonyl, acryloyl, propioloyl,
cyclopentyl, cyclohexyl,
in each case doubly attached propanediyl, ethyleneoxy, each of which is optionally mono- to tetrasubstituted by identical or different substituents from the group consisting of fluorine, chlorine, oxo, methyl and trifluoromethyl,
or a grouping 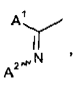 where
A¹ represents hydrogen, methyl or hydroxyl and
A² represents hydroxy, methoxy, ethoxy, amino, methylamino, phenyl or benzyl, and also
phenyl, phenoxy, phenylthio, benzoyl, benzoylethenyl, cinnamoyl, benzyl, phenylethyl, phenylpropyl, benzyloxy, benzylthio, 5,6-dihydro-1,4,2-dioxaxin-3-ylmethyl, triazolylmethyl, benzoxazol-2-ylmethyl, 1,3-dioxan-2-yl, benzimidozol-2-yl, dioxol-2-yl, oxadiazolyl, each of which is optionally mono- to trisubstituted in the ring moiety by halogen and/or straight-chain or branched alkyl or alkoxy having 1 to 4 carbon atoms,
X represents fluorine,
L¹, L², L³ and L⁴ each represent hydrogen,
and
L⁵ represents one of the groups below: 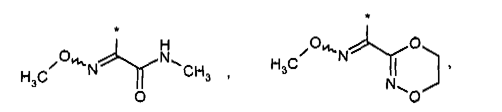
where * denotes the point of attachment to the phenyl radical.

9. Process according to at least one of Claims 1 to 8, **characterized in that** the compound of the formula (1-3) is obtained as a reaction product

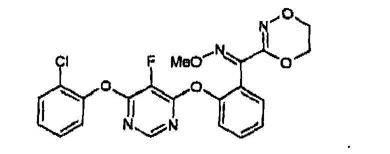

10. Process according to at least one of Claims I to 9, **characterized in that** the base used in the first step is potassium carbonate or sodium carbonate.

11. Process according to at least one of Claims 1 to 10, **characterized in that** the solvent used in the first step is methyl isobutyl ketone.

12. Process according to at least one of Claims 1 to 11, **characterized in that** the first step is carried out at temperatures of 40 - 80°C.

13. Process according to at least one of Claims 1 to 12, **characterized in that** the base used in the second step is potassium carbonate or sodium carbonate.

14. Process according to at least one of Claims 1 to 13, **characterized in that** the solvent used in the second step is methyl isobutyl ketone.

15. Process according to at least one of Claims 1 to 14, **characterized in that** the second step is carried out at temperatures of 50 - 80°C.

## Revendications

1. Procédé pour la préparation de composés de formule générale (I), 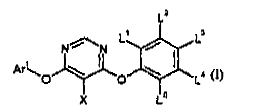 dans laquelle
Ar¹ représente un groupe hétérocyclyle ayant de 3 à 7 chaînons formant le cycle, éventuellement substitué par halogène ou par alkyle, alcoxy, halogénoalkyle, halogénoalcoxy ayant chacun de 1 à 4 atomes de carbone ;
ou représente un groupe phényle ou naphtyle portant éventuellement chacun un à quatre substituants identiques ou différents, les éventuels substituants étant choisis de préférence dans la liste suivante :
halogène, cyano, nitro, formule, carboxy, carbamoyle, thiocarbamoyle ;
alkyle, oxoalkyle, alcoxy, alcoxyalkyle, alkylthio-alkyle, dialcoxyalkyle, alkylthio, alkylsulfinyle ou alkylsulfonyle chacun à chaîne droite ou ramifiée, ayant chacun de 1 à 8 atomes de carbone ;
alcényle ou alcényloxy chacun à chaîne droite ou ramifiée, ayant chacun de 2 à 6 atomes de carbone ;
halogénoalkyle, halogénoalcoxy, halogénoalkylthio, halogénoalkylsulfinyle ou halogénoalkylsulfonyle chacun à chaîne droite ou ramifiée, ayant chacun de 1 à 6 atomes de carbone et de 1 à 13 atomes d'halogène identiques ou différentes ; halogénoalcényle ou halogénoalcényloxy chacun à chaîne droit ou ramifiée, ayant chacun de 2 à 6 atomes de carbone et de 1 à 11 atomes d'halogène identiques ou différents ;
dialkylamino à chaîné droite ou ramifiée,
alkylcarbonyle, alkylcarbonyloxy, alcoxycarbonyle, alkylaminocarbonyle, dialkylaminocarbonyle, arylalkylaminocarbonyle, dialkylaminocarbonyloxy, alcénylcarbonyle ou alcynylcarbonyle, ayant de 1 à 6 atomes de carbone dans les chaînes hydrocarbonées respective ;
cycloalkyle ou cycloalkyloxy ayant chacun de 3 à 6 atomes de carbone ;
alkylène ayant 3 ou 4 atomes de carbone, oxyalkylène ayant 2 ou 3 atomes de carbon ou dioxoalkylène ayant 1 ou 2 atomes de carbone, chacun lié deux fois, chacun portant éventuellement un à quatre substituant, identiques ou différents, fluoro, chloro, oxo, méthyle, trifluorométhyle ou éthyle ;
ou un groupement 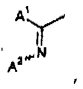 dans lequel
A¹ représente un atome d'hydrogène, un groupe hydroxy ou alkyle ayant de 1 à 4 atomes de carbone ou cycloalkyle ayant de 1 à 6 atomes de carbone et
A² représente un groupe hydroxy, méthoxy, éthoxy, amino, méthylamino, phényle, benzoyle ou un groupe alkyle ou alcoxy ayant de 1 à 4 atomes de carbone, chacun éventuellement substitué par cyano, alcoxy, alkylthio, alkylamino, dialkylamino ou phényle, ou un groupe alcényloxy ou alcynylthio ayant chacun de 2 à 4 atomes de carbone,
ainsi qu'un groupe phényle, benzoyle, benzoyléthényle, cinnamoyle, hétérocyclyle ou phénylalkyle, phénylalkyloxy ou hétérocyclylalkyle, ayant chacun de 1 à 3 atomes de carbone dans les fragments alkyle respectifs, chacun éventuellement une à trois fois substitué sur le fragment cyclique par halogène et/ou alkyle ou alcoxy à chaîne droite ou ramifiée, ayant de 1 à 4 atomes de carbone,
X représente un atome de fluor ou de chlore,
L¹, L², L³, L⁴ et L⁵ sont identiques ou différents et représentent chacun, indépendamment les uns des autres, un atome d'hydrogène ou d'halogène, un groupe cyano, nitro, formule, alkylcarbonyle, alcoxycarbonyle, aminocarbonyle, alkylaminocarbonyle, dialkylaminocarbonyle ayant chacun de 1 à 6 atomes de carbone, un groupe alkyle, alcoxy, alkylthio, alkylsulfinyle ou alkylsulfonyle ayant chacun de 1 à 6 atomes de carbone, chacun éventuellement substitué par 1 à 5 atomes d'halogène,
ou
L¹, L², L³ et L⁴ sont identiques ou différents et représentent chacun, indépendamment les uns des autres, un atome d'hydrogène ou d'halogène, un groupe cyano, nitro, formyle, alkylcarbonyle, alcoxycarbonyle, aminocarbonyle, alkylaminocarbonyle, dialkylaminocarbonyle ayant chacun de 1 à 6 atomes de carbone, un groupe alkyle, alcoxy, alkylthio, alkylsulfinyle ou alkylsulfonyle ayant chacun de 1 à 6 atomes de carbone, chacun éventuellement substitué par 1 à 5 atomes d'halogène,
et
L⁵ représente l'un des groupes suivants : 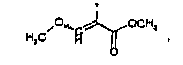 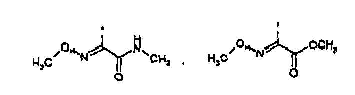 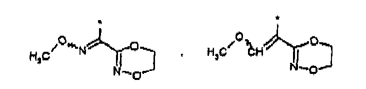 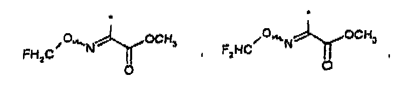 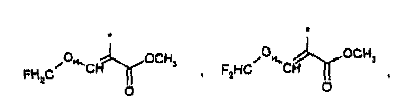 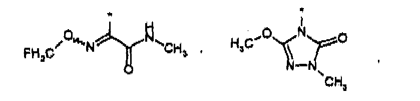 où * désigne la position de liaison au radical phényle,
et où les radicaux
Ar¹ et 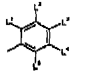 sont différentes,
**caractérisé en ce qu'**on fait réagir des dérivés de 4,6-dichloropyrimidine de formule générale (II), 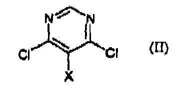 dans laquelle
X a la signification indiquée plus haut,
a) d'abord dans une première étape avec des composés de formule générale (III),
Ar¹- OH, (III),
dans laquelle
Ar¹ a la signification indiquée plus haut,
éventuellement en présence d'un diluant et éventuellement en présence d'un accepteur d'acide,
et on fait réagir les composés de formule (IV) qui en résultent, 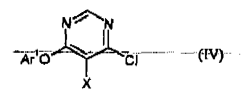 dans laquelle
Ar¹ et X ont les significations indiquées plus haut,
puis dans une deuxième étape avec des composés de formule générale (V), 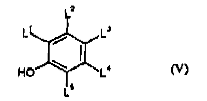 dans laquelle
L¹, L², L³, L⁴ et L⁵ ont les significations indiquées plus haut,
éventuellement en présence d'un solvant, en présence d'une base et avec addition de 2 à 20 % en moles de 1,4-diazabicyclo[2.2.2]octane (DABCO),
ou
b) d'abord dans une première étape avec des composés de formule générale (V), 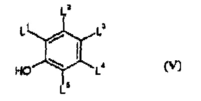 dans laquelle
L¹, L², L³, L⁴ et L⁵ ont les significations indiquées plus haut,
éventuellement en présence d'un diluant et éventuellement en présence d'un accepteur d'acide,
et on fait réagir les composés de formule (VI) qui en résultent 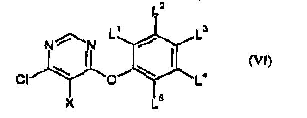 dans laquelle
X, L¹, L², L³, L⁴ et L⁵ ont les significations indiquées plus haut,
puis, dans une deuxième étape avec des composés de formule générale (III)
Ar¹ -OH, (III)
dans laquelle
Ar¹ a la signification indiquée plus haut,
éventuellement en présence d'un solvant, en présence d'une base et avec addition de 2 à 20 % en moles de 1,4-diazabicyclo[2.2.2]octane (DABCO).

2. Procédé selon la revendication 1, **caractérisé en ce que** le procédé est effectué pour la préparation des composés de formule (I) en tant que procédé en un seul récipient.

3. Procédé selon la revendication 1 ou 2, **caractérisé en ce qu'**on utilise de 1 à 4 moles du dérivé de 4,6-dichloropyrimidine de formule (II) par mole des composés de formule (III),

4. Procédé selon la revendication 1 ou 2, **caractérisé en ce qu'**on utilise de 1 à 4 moles du dérivé de 4,6-dichloropyrimidine de formule (II) par mole des composés de formule (V).

5. Procédé selon au moins l'une des revendications 1 à 4, **caractérisé en ce qu'**on utilise de 0,8 à 4 moles des composés de formule (V) par mole des composés de formule (IV).

6. Procédé selon au moins l'une des revendications 1, à 4, **caractérisé en ce qu'**on utilise de 0,8 à 4 moles des composés de formule (III) par mole des composés de formule (VI).

7. Procécé selon au moins l'une des revendications 1 à 6, **caractérisé en ce que**
Ar¹ représente un groupe thiényle, pyridyle ou furyle éventuellement substitué par méthyle, éthyle, méthoxy, éthoxy, trifluorométhyle ou trifluorométhoxy ;
ou représente un groupe phényle éventuellement portant chaque fois un à quatre substituant identiques ou différent, les éventuels substituants étant choisis de préférence dans la liste suivante :
fluore, chlore, brome, iode, cyano, nitro, formyle carboxy, carbamoyle, thiocarbamoyle,
méthyle, éthyle, n- ou isopropyle, n-, iso-, sec- ou tert-butyle, 1-, 2-, 3-, néopentyle, 1-, 2-, 3-, 4-(2-méthylbutyle), 1-, 2-, 3-hexyle, 1-, 2-, 3-, 4-, 5-(2-méthylpentyle), 1-, 2-, 3-(3-méthylpentyle), 2-éthylbutyle, 1-, 3-, 4-(2,2-diméthylbutyle), 1-, 2-(2,3-diméthylbutyle) , 3-oxobutyle, méthoxyméthyle, diméthoxyméthyle,
méthoxy, éthoxy, n- ou isopropoxy,
méthylthio, éthylthio, n- ou isopropylthio, méthylsulfinyle, éthylsulfinyle, méthylsulfonyle ou éthylsulfonyle,
vinyle, allyle, 2-méthylallyle, propén-1-yle, crotonyle, propargyle, vinyloxy, allyloxy, 2-méthylallyloxy, propén-1-yloxy, crotonyloxy, propargyloxy ;
trifluorométhyle, trifluoroéthyle,
difluorométhoxy, trifluorométhoxy, difluorochlorométhoxy, trifluoroéthoxy, difluorométhylthio, trifluorométhylthio, difluorochlorométhylthio, trifluorométhylsulfinyle ou trifluorométhylsulfonyle, diméthylamino, diéthylamino,
acétyle, propionyle, méthoxycarbonyle, éthoxycarbonyle, méthylaminocarbonyle, éthylaminocarbonyle, diméthylaminocarbonyle, diéthylaminocarbonyle diméthylaminocarbonyloxy, diéthylaminocarbonyloxy, benzylaminocarbonyle, acryloyle, propioloyle,
cyclopentyle, cyclohexyle,
propanediyle, éthylène-oxy, chacun à deux liaisons, portant éventuellement chacun une à quatre substituants, identiques ou différents, fluoro, chloro, oxo, méthyle ou trifluorométhyle,
ou un groupement 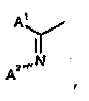 où
A¹ représente un atome d'hydrogène, un groupe méthyle ou hydroxy et
A² représente un groupe hydroxy, méthoxy, éthoxy, amino, méthylamino, phényle ou benzoyle, ainsi que
un groupe phényle, benzoyle, benzoyléthényle, cinnamoyle, benzyle, phényléthyle, phénylpropyle, benzyloxy, 5,6-dihydro-1,4,2-dioxazin-3-ylméthyle, triazolylméthyle, benzoxazol-2-ylméthyle, 1,3-dioxann-2-yle, benzimidazol-2-yle, dioxol-2-yle, oxadiazolyle, chacun éventuellement une à trois fois substitué sur le fragment cyclique par halogène et/ou alkyle ou alcoxy à chaîne droite ou ramifiée, ayant de 1 à 4 atomes de carbone,
X représente un atome de fluor,
L¹, L², L³ et L⁴ sont identiques ou différents et représentent chacun, indépendamment les uns des autres, un atome d'hydrogène, de fluor, chlore, brome, un groupe cyano, nitro, acétyle, propionyle, méthoxycarbonyle, éthoxycarbonyle, méthylaminocarbonyle, éthylaminocarbonyle, diméthylaminocarbonyle, diéthylaminocarbonyle, méthyle, éthyle, n- ou isopropyle, n-, iso-, sec-ou tert-butyle, méthoxy, éthoxy, n- ou isopropoxy, méthylthio, éthylthio, méthylsulfinyle, éthylsulfinyle, méthylsulfonyle ou éthylsulfonyle, trifluorométhyle, trifluoroéthyle, difluorométhoxy, trifluorométhoxy, difluorochlorométhoxy, trifluoroéthoxy, difluorométhylthio, difluorochlorométhylthio, trifluorométhylthio, trifluorométhylsulfinyle ou trifluorométhylsulfonyle, et
L⁵ représente l'un des groupes suivants : 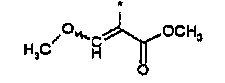 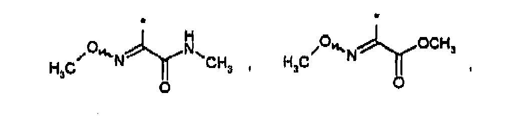 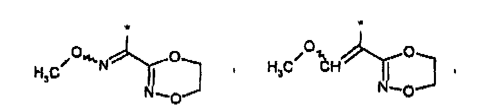 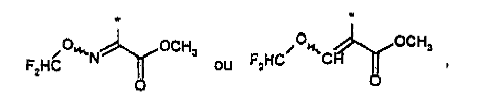 où * désigne la position de liaison au radical phényle.

8. Procédé selon au moins l'une des revendications 1 à 7, **caractérisé en ce que**
Ar¹ représente un groupe thiényle, pyridyle ou furyle, éventuellement substitué par méthyle, éthyle, méthoxy, éthoxy, trifluorométhyle ou trifluorométhoxy ;
ou représente un groupe phényle éventuellement portant chaque fois un à quatre substituants identiques ou différents, les éventuels substituants étant choisis de préférence dans la liste suivante :
fluore, chlore, brome, iode, cyano, nitro, formyle carboxy, carbamoyle, thiocarbamoyle,
méthyle, éthyle, n- ou isopropyle, n-, iso-, sec- ou tert-butyle, 1-, 2-, 3-, néopentyle, 1-, 2-, 3-, 4-(2-méthylbutyle), 1-, 2-, 3-hexyle, 1-, 2-, 3-, 4-, 5-(2-méthylpentyle), 1-, 2-, 3-(3-méthylpentyle), 2-éthyl-butyle, 1-, 3-, 4-(2,2-diméthylbutyle), 1-, 2-(2,3-diméthylbutyle), 3-oxobutyle, méthoxyméthyle, diméthoxyméthyle,
méthoxy, éthoxy, n- ou isopropoxy,
méthylthio, éthylthio, n- ou isopropylthio, méthylsulfinyle, éthylsulfinyle, méthylsulfonyle ou éthylsulfonyle,
vinyle, allyle, 2-méthylallyle, propén-1-yle, crotonyle, propargyle, vinyloxy, allyloxy, 2-méthylallyloxy, propén-1-yloxy, crotonyloxy, propargyloxy ;
trifluorométhyle, trifluoroéthyle,
difluorométhoxy, trifluorométhoxy, difluorochlorométhoxy, trifluoroéthoxy, difluorométhylthio, trifluorométhylthio, difluorochlorométhylthio, trifluorométhylsulfinyle ou trifluorométhylsulfonyle, diméthylamino, diéthylamino,
acétyle, propionyle, méthoxycarbonyle, éthoxycarbonyle, méthylaminocarbonyle, éthylaminocarbonyle, diméthylaminocarbonyle, diéthylaminocarbonyle diméthylaminocarbonyloxy, diéthylaminocarbonyloxy, benzylaminocarbonyle, acryloyle, propioloyle,
cyclopentyle, cyclohexyle,
propanediyle, éthylène-oxy, chacun à deux liaisons, portant éventuellement chacun un à quatre substituant, identiques ou différents, fluoro, chloro, oxo, méthyle ou trifluorométhyle,
ou un groupement 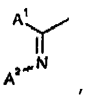 où
A¹ représente un atome d'hydrogène, un groupe méthyle ou hydroxy et
A² représente un groupe hydroxy, méthoxy, éthoxy, amino, méthylamino, phényle ou benzyle, ainsi que
un groupe phényle, phénoxy, phénylthio, benzoyle, benzoyléthényle, cinnamoyle, benzyle, phényléthyle, phénylpropyle, benzyloxy, benzylthio, 5,6-dihydro-1,4,2-dioxazin-3-ylméthyle, triazolylméthyle, benzoxazol-2-ylméthyle, 1,3-dioxann-2-yle, benzimidazol-2-yle, dioxol-2-yle, oxadiazolyle, chacun éventuellement une à trois fois substitué sur le fragment cyclique par halogène et/ou alkyle ou alcoxy à chaîne droite ou ramifiée, ayant de 1 à 4 atomes de carbone,
X représente un atome de fluor, L¹, L², L³ et L⁴ représentant des atomes d'hydrogène, et
L⁵ représente l'un des groupes suivants : 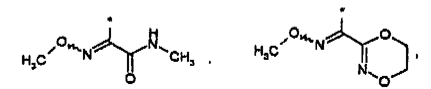 où * désigne la position de liaison au radical phényle.

9. Procécé selon au moins l'une des revendications 1 à 8, **caractérisé en ce qu'**on obtient comme produit de réaction le composé de formule (I-3)

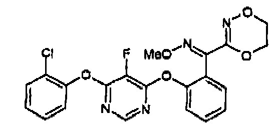

10. Procédé selon au moins l'une des revendications 1 à 9, **caractérisé en qu'**on utilise comme base dans la première étape du carbonate de sodium ou de potassium.

11. Procécé selon au moins l'une des revendications 1 à 10, **caractérisé en qu'**on utilise comme solvant dans la première étape la méthylisobutylcétone.

12. Procédé selon au moins l'une des revendications 1 à 11, **caractérisé en que** dans la première étape on opère à des températures de 40 - 80 °C.

13. Procédé selon au moins l'une des revendications 1 à 12, **caractérisé en qu'**on utilise comme base dans la deuxième étape du carbonate de sodium ou de potassium.

14. Procédé selon au moins l'une des revendications 1 à 13, **caractérisé en qu'**on utilise comme solvant dans la deuxième étape la méthylisobutylcétone.

15. Procédé selon au moins l'une des revendications 1 à 14, **caractérisé en que** dans la deuxième étape on opère à des températures de 50 - 80 °C.
